# EUROPÄISCHE PATENTSCHRIFT

(11) **EP 0 694 530 B1**
(45) Veröffentlichungstag und Bekanntmachung des Hinweises auf die Patenterteilung: **23.08.2000**
(21) Anmeldenummer: 95111158.2
(22) Anmeldetag: 17.07.1995
(51) Int. Cl.: C07C 253/30, C07C 255/50, C07D 239/30, C07B 37/04, C09K 19/00

(54) **Verfahren zur Kreuzkupplung von aromatischen Borverbindungen mit aromatischen Halogenverbindungen oder Perfluoralkylsulfonaten**
Process for cross-coupling of aromatic boron compounds with aromatic halogen compounds or perfluoroalkyl sulphonates
Procédé de couplage croisé de composés aromatiques du bore avec des composés aromatiques halogénés ou de sulphates de peralkyl

(30) Priorität: 28.07.1994 DE 4426671
(43) Veröffentlichungstag der Anmeldung: 31.01.1996
(73) Patentinhaber: Aventis Research & Technologies GmbH & Co. KG, 65926 Frankfurt am Main (DE)
(72) Erfinder: Haber, Steffen, Dr., D-76726 Germersheim (DE); Manero, Javier, Dr., D-65931 Frankfurt (DE)
(74) Vertreter: Isenbruck, Günter, Dr.

(56) Entgegenhaltungen:
- WO-A-94/00423
- DE-A- 4 236 103
- DE-A- 4 340 490
- US-A- 5 043 510
- JOURNAL OF THE AMERICAN CHEMICAL SOCIETY, Bd. 112, Nr. 11, 23.Mai 1990 Seiten 4324-4330, XP 000561945 CASALNUOVO A L ET AL 'PALLADIUM-CATALYZED ALKYLATIONS IN AQUEOUS MEDIA'
- SYNLETT, Nr. 9, 1.September 1992 Seiten 715-717, XP 000561946 GENET J P ET AL 'PALLADIUM-CATALYZED CROSS-COUPLING REACTIONS IN A HOMOGENEOUS AQUEOUS MEDIUM'

## Beschreibung

Die Erfindung betrifft ein Verfahren zur Herstellung mehrkerniger aromatischer Verbindungen durch Kreuzkupplung von aromatischen Borverbindungen mit aromatischen Halogenverbindungen oder Perfluoralkylsulfonaten in einem Zweiphasensystem unter Palladiumkatalyse in Anwesenheit von wasserlöslichen Komplexliganden.

Die palladiumkatalysierte Kreuzkupplungsreaktion von aromatischen Borverbindungen, wie Boronsäuren und deren Derivaten oder Boranen, und aromatischen Halogenverbindungen oder - Perfluoralkylsulfonaten (siehe z.B. N. Miyaura, T. Yanagi, A. Suzuki in Synthetic Communications 11 (1981) 513; EP-A 0 470 795 und EP-A 0 354 434) wird seit einigen Jahren in steigendem Umfang in vielen Bereichen der organischen Synthese genutzt.

Bei den zitierten Verfahren handelt es sich um homogen katalysierte Prozesse unter Verwendung von Pd(0)-Komplexen, insbesondere Tetrakis-(triphenylphosphan)-palladium(0). Die Anwesenheit von phosphorhaltigen Komplexliganden steigert im allgemeinen Ausbeuten und Selektivität der Reaktion erheblich.

Ein Nachteil dieser Verfahren liegt jedoch unter anderem bei den hohen Katalysatorkosten, die eine wirtschaftliche Überführung der Prozesse in einen größeren Produktionsmaßstab (kg, t) schwierig machen. Zudem wird eine Kontamination des Produktes und des Abfalls mit Phosphorverbindungen beobachtet, was insbesonders bei Wirkstoffen ein Nachteil sein kann.

Es ist auch bekannt, wasserlösliche Palladiumkomplexe für die obengenannten Kupplungsreaktionen einzusetzen und in rein wäßrigen - oder Zweiphasensystemen aus organischer - und Wasserphase zu arbeiten (siehe z.B. US 5,043,510; J.P. Genet et al., Synlett 1992, 715). Dabei werden wasserlösliche Phosphanliganden, wie Triphenylphosphano-3,3',3"-trisulfonattrinatriumsalz (tppts), verwendet, um den wasserlöslichen Palladiumkomplex zu erhalten. Es ist jedoch beschrieben (A.L. Casalnuovo und J.C. Calabrese, J. Am. Chem. Soc. 112 (1990) 4324), daß die Ausbeuten im Zweiphasensystem deutlich niedriger als im Einphasensystem liegen.

Zudem leiden diese Prozesse unter den oben beschriebenen Nachteilen der Katalysatorkosten und der Verunreinigung des Produkts mit den Komplexliganden.

Neben den bereits vorstehend aufgeführten Verfahrensvarianten sind in der DE-A 43 40 490 auch schon lipophile (also gerade nicht wasserlösliche) Phosphinliganden in einer speziellen Kreuzkopplungsreaktion mit 2,6-Difluor-4-chlortrifluormethoxybenol beschrieben worden. Das organische Lösungsmittel (z.B. Dichlormethan) zur Erzeugung der organischen Phase wird erst nach Beendigung der eigentlichen Reaktion zugegeben. Auch die Beschreibung der WO 94/00423 nennt zwar Lösungsmittel-Wasser-Gemische und z.B. Palladium als Katalysator, aber die dort aufgeführten Promotoren und das Reaktionssystem führen nicht zu dem speziellen Reaktions-/Aufarbeitungsgleichgewicht des erfindungsgemäßen Verfahrens.

Es sind Verfahren entwickelt worden, die das Problem der Katalysatorkosten durch den Einsatz von Palladiummetall als heterogenen Katalysator verringern. Das Vorhandensein einer festen Phase in der Reaktionsmischung ist jedoch nicht immer erwünscht.

Aufgabe der vorliegenden Erfindung war es daher, ein ökonomisch günstiges Verfahren zur Kupplung von aromatischen Borverbindungen mit aromatischen Perfluoralkylsulfonaten bereitzustellen, das die Kupplungsprodukte in sehr hoher Reinheit und guter Ausbeute liefert, ohne daß die Reaktion in Gegenwart einer festen Phase durchgeführt werden muß.

Es wurde nun überraschend gefunden, daß bei Umsetzung von aromatischen Borverbindungen, wie Boronsäuren, mit aromatischen Halogenverbindungen oder - Perfluoralkylsulfonaten in einem zweiphasigen Reaktionsmedium in Gegenwart einer Base, katalytischer Mengen einer in organischen Lösemitteln lösliche Palladiumverbindung und mindestens eines wasserlöslichen Komplexliganden mehrkernige aromatische Verbindungen in hervorragenden Ausbeuten und sehr hohen Reinheiten erhalten werden.

Gegenstand der Erfindung ist somit ein Verfahren zur Herstellung mehrkerniger aromatischer Verbindungen durch Kreuzkupplung von aromatischen Borverbindungen mit aromatischen Halogenverbindungen oder Perfluoralkylsulfonaten unter Palladiumkatalyse in Gegenwart mindestens eines wasserlöslichen Komplexliganden in einem Wasser enthaltenden Reaktionsmedium. Das erfindungsgemäße Verfahren ist dann dadurch gekennzeichnet, daß das Reaktionsmedium eine wäßrige und eine organische Phase bildet und das Palladium in Form einer in der organischen Phase löslichen Palladiumverbindung aus der Gruppe Palladiumketonate, Palladiumacetylacetonate, Nitrilpalladiumhalogenide, Olefinpalladiumhalogenide, Palladiumhalogenide, Allylpalladiumhalogenide und Palladiumbiscarboxylate zugesetzt wird, und als wasserlöslicher Ligand mindestens eine Verbindung aus der Gruppe Phosphole, Phosphite, Phosphonigsäureester, Phosphinigsäureester, Phosphole, Bipyridine, Phenanthroline, Porphysine und Alizarine verwendet wird.

Die erfindungsgemäße Reaktion verläuft chemoselektiv, so daß selbst elektrophile Gruppen, wie Ester oder Nitrile, den Verlauf der Reaktion nicht beeinträchtigen.

Durch das erfindungsgemäße Verfahren lassen sich mehrkernige aromatische Verbindungen ökonomisch in sehr guten Ausbeuten und gleichzeitig sehr hoher Reinheit, insbesondere ohne Verunreinigung durch die Komplexliganden, herstellen.

Das erfindungsgemäße Verfahren wird in einem Mehrphasensystem aus einer wäßrigen Phase und einer organischen Phase durchgeführt. Die wäßrige Phase kann dabei neben Wasser auch ein oder mehrere wasserlösliche organische Lösungsmittel enthalten.

Zur Durchführung des erfindungsgemäßen Verfahrens werden die aromatische Borverbindung, die aromatische Halogenverbindung oder das Perfluoralkylsulfonat, die Base, die katalytische Menge der in organischen Lösemitteln löslichen Palladiumverbindung und der wasserlösliche Ligand in ein Mischung aus Wasser und einem oder mehreren inerten organischen Lösungsmitteln gegeben und bei einer Temperatur von 0°C bis 200°C, bevorzugt bei 30°C bis 170°C, besonders bevorzugt bei 50°C bis 150°C, insbesondere bevorzugt bei 60°C bis 120°C, für einen Zeitraum von 1 h bis 100 h, bevorzugt 5 h bis 70 h, besonders bevorzugt 5 h bis 50 h, gerührt.

Das erfindungsgemäße Verfahren eignet sich vorzugsweise zur Herstellung von Produkten, die aus der wäßrigen in eine organische Phase extrahierbar sind.

Die Aufarbeitung erfolgt dann nach bekannten, dem Fachmann geläufigen Verfahren.

Um eine Verunreinigung des Produktes mit Palladium zu vermeiden, kann beispielsweise nach Reaktionsende der Reaktionsmischung soviel Komplexligand zugesetzt werden, daß das gesamte Palladium in die wäßrige Phase gezogen wird. Gegebenenfalls kann aber auch nach Abschluß der Reaktion ein wasserlöslicher Komplexbilder zugesetzt werden, der nicht mit dem in der Reaktion eingesetzten identisch ist, um das Palladium vollständig in die wäßrige Phase überzuführen.

Natürlich kann das Palladium auch gegebenenfalls durch chromatographische Methoden oder durch Ausfällung, beispielsweise als Sulfid, abgetrennt werden.

Der wasserlösliche, typischerweise phosphorhaltige Komplexligand wird durch Trennung von wäßriger- und organischer Phase bereits vollständig vom Produkt abgetrennt.

Üblicherweise wird zur weiteren Aufarbeitung nach der Phasentrennung das Rohprodukt vom Lösungsmittel befreit und anschließend nach dem jeweiligen Produkt angemessenen Methoden, z.B. durch Umkristallisation, Destillation, Sublimation, Zonenschmelzen, Schmelzkristallisation oder Chromatographie, weiter aufgereinigt.

Für das erfindungsgemäße Verfahren geeignete organische Lösungsmittel, die in dem Reaktionsmedium eine organische Phase ausbilden, sind beispielsweise Ether, wie Diethylether, Dimethoxymethan, Diethylenglykoldimethylether, Diisopropylether, tert.-Butylmethylether, Kohlenwasserstoffe, wie Hexan, iso-Hexan, Heptan, Cyclohexan, Benzol, Toluol, Xylol, höhere, mit Wasser nicht beliebig mischbare Alkohole, wie 1-Butanol, 2-Butanol, tert.-Butanol, Amylalkohol, Ketone, wie iso-Butylmethylketon, Amide, wie Dimethylacetamid, N-Methylpyrrolidon, Nitrile, wie Butyronitril, und Mischungen derselben.

Bevorzugte organische Lösungsmittel sind Ether, wie Diethylether, Dimethoxyethan, Diethylenglykoldimethylether, Diisopropylether, Kohlenwasserstoffe, wie Hexan, Heptan, Cyclohexan, Benzol, Toluol, Xylol, Alkohole, wie 1-Butanol, 2-Butanol, tert.-Butanol, Ketone, wie iso-Butylmethylketon, Amide, wie N-Methylpyrrolidon, und Mischungen derselben.

Besonders bevorzugte Lösungsmittel sind Kohlenwasserstoffe, z. B. Cyclohexan, Benzol, Toluol, Xylol und Mischungen derselben.

In einer besonders bevorzugten Variante werden bei dem erfindungsgemäßen Verfahren Wasser, ein oder mehrere in Wasser unlösliche und ein oder mehrere in Wasser lösliche Lösungsmittel eingesetzt.

Bevorzugte mit der wäßrigen Phase mischbare organische Cosolvenzien sind Nitrile, wie Acetonitril, Formamide, wie DMF, niedere Alkohole, wie Methanol und Ethanol, Sulfoxide, wie DMSO, und cyclische Ether, wie THF oder Dioxan.

Bevorzugte Reaktionsmedien aus Wasser, wasserlöslichem organischen Lösungsmittel und wasserunlöslichem organischen Lösungsmittel sind Mischungen aus Wasser, Toluol und Ethanol, Wasser, Toluol und Tetrahydrofuran und Wasser, Toluol und Acetonitril, vorzugsweise jeweils im Volumenverhältnis 1:2:1.

Basen, die bei dem erfindungsgemäßen Verfahren üblicherweise Verwendung finden, sind Alkali- und Erdalkalimetallhydroxide, Alkali- und Erdalkalimetallcarbonate, Alkalimetallhydrogencarbonate, Alkali- und Erdalkalimetallacetate, Alkali- und Erdalkalimetallalkoholate, sowie primäre, sekundäre und tertiäre Amine.

Besonders bevorzugt sind Alkali- und Erdalkalimetallhydroxide, Alkali- und Erdalkalimetallcarbonate und Alkalimetallhydrogencarbonate. Insbesondere bevorzugt sind Alkalimetallhydroxide, wie Natriumhydroxid und Kaliumhydroxid, sowie Alkalimetallcarbonate und Alkalimetallhydrogencarbonate, wie Lithiumcarbonat, Natriumcarbonat und Kaliumcarbonat.

Die Base wird bei dem erfindungsgemäßen Verfahren bevorzugt mit einem Anteil von 100 bis 1000 Mol-%, besonders bevorzugt 100 bis 500 Mol-%, ganz besonders bevorzugt 150 bis 400 Mol-%, insbesondere 180 bis 250 Mol-%, bezogen auf die aromatische Borverbindung, eingesetzt.

Als Katalysator dienen die in organischen Lösungsmitteln löslichen Palladiumverbindungen, bevorzugt Palladiumketonate, Palladiumacetylacetonate, bis-η²-Olefinpalladiumdihalogenide, Palladium(II)halogenide, η-³-Allylpalladiumhalogenid Dimere und Palladiumbiscarboxylate, ganz besonders bevorzugt Bis(dibenzylidenaceton)palladium(0) [Pd(dba)₂)], Pd(dba)₂•CHCI₃, Palladiumbisacetylacetonat, Bis(benzonitril)palladiumdichlorid, PdCl₂, Na₂PdCl₄, Dichlorobis(dimethylsulfoxid)palladium(II), Bis(acetonitril)palladiumdichlorid, Palladium-II-acetat, Palladium-II-propionat, Palladium-II-butanoat und (1c,5c-Cyclooctadien)palladiumdichlorid.

Der Palladiumkatalysator wird bei dem erfindungsgemäßen Verfahren mit einem Anteil von 0,001 bis 10 Mol-%, bevorzugt 0,01 bis 5 Mol-%, besonders bevorzugt 0,05 bis 3 Mol-%, insbesonders bevorzugt 0,1 bis 1,5 Mol-%, bezogen auf die aromatische Halogenverbindung oder das Perfluoralkylsulfonat, eingesetzt.

Die im erfindungsgemäßen Verfahren geeigneten wasserlösliche Liganden enthalten beispielsweise Sulfonsäuresalz- und/oder Sulfonsäurereste und/oder Carbonsäuresalz- und/oder Carbonsäurereste und/oder Phosphonsäuresalz und/oder Phosphonsäurereste und/oder Phosphoniumgruppen und/oder Peralkylammoniumgruppen und/oder Hydroxygruppen und/oder Polyethergruppen mit geeigneter Kettenlänge.

Erfindungsgemäß eingesetzt werden mit den obigen Gruppen substituierte Phosphane, wie Trialkylphosphane, Tricycloalkylphosphane, Triarylphosphane, Dialkylarylphosphane, Alkyldiarylphosphane und Heteroarylphosphane wie Tripyridylphosphan und Trifurylphosphan, wobei die drei Substituenten am Phosphor gleich oder verschieden, chiral oder achiral sein können und wobei einer oder mehrere der Liganden die Phosphorgruppen mehrerer Phosphane verknüpfen können und wobei ein Teil dieser Verknüpfung auch ein oder mehrere Metallatome sein können, Phosphite, Phosphinigsäureester und Phosphonigsäureester, Phosphole, wie Dibenzophosphole und Phosphoratome enthaltende cyclische bzw. oligo- und polycyclische Verbindungen; weiterhin sind dies Bipyridine, Phenanthroline, Porphyrine und Alizarine, die mit den obengenannten Gruppen modifiziert sind.

Bevorzugt eingesetzte wasserlösliche Phosphane sind solche der allgemeinen Formeln (I) bis (VII), 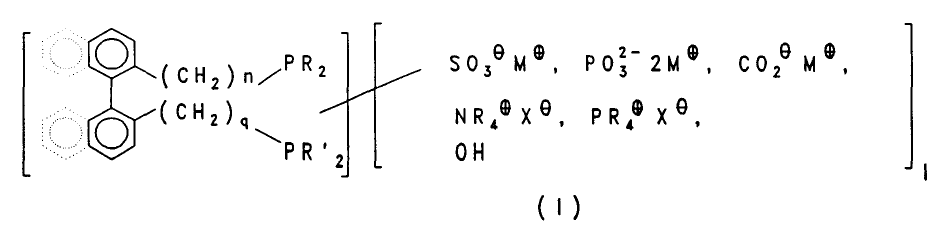 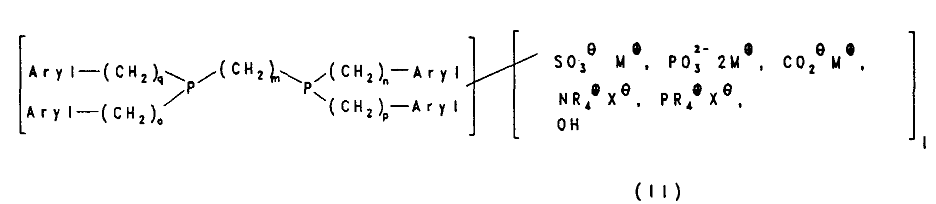 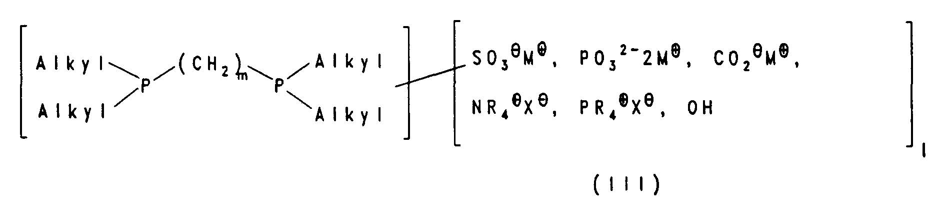 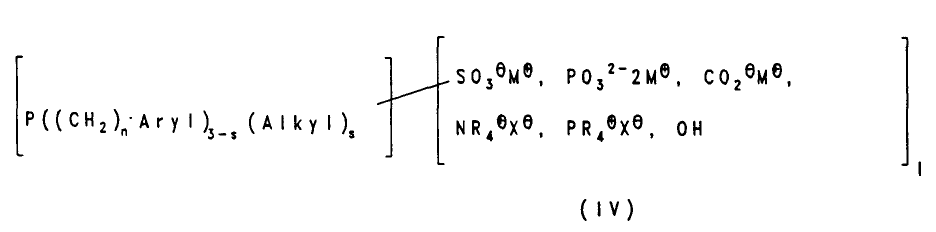 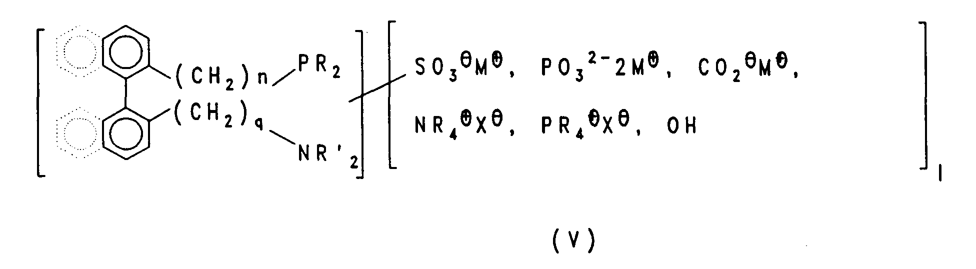 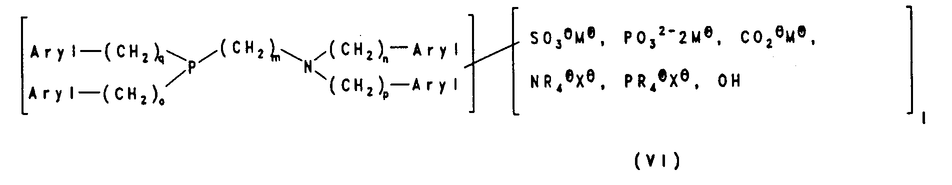 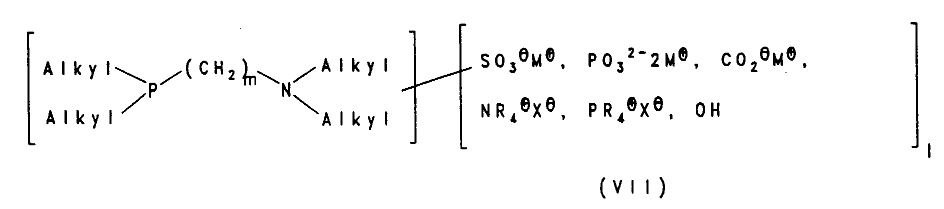 wobei die Symbole und Indizes folgende Bedeutungen haben:
- Aryl:: eine Phenyl- oder Naphthylgruppe, die auch einen oder mehrere Substituenten R tragen kann;
- Alkyl:: eine geradkettige oder verzweigte Alkylgruppe mit 1 bis 8 Kohlenstoffatomen;
- R,R':: Alkyl, Aryl oder Aralkyl mit 1 bis 18 Kohlenstoffatomen;
- M:: Alkali-, Erdalkalimetall oder NR₄;
- X:: Halogen, BF₄, PF₆, OSO₂CF₃, 1/2[SO₄];
- l,m:: 1 bis 8;
- n,o,p,q:: 0, 1 bis 8;
- s:: 0, 1 bis 3.

Im folgenden sind Beispiele für besonders bevorzugte wasserlösliche Komplexliganden aufgeführt:
(R hat dabei, wenn nicht anders vermerkt, die in den Formeln (I) bis (VII) angegebenen Bedeutungen)
1. Sulfonierte Phosphane 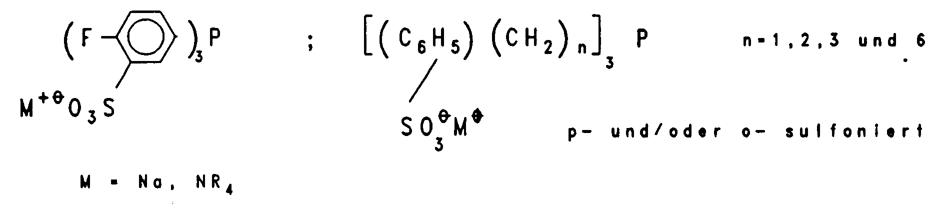 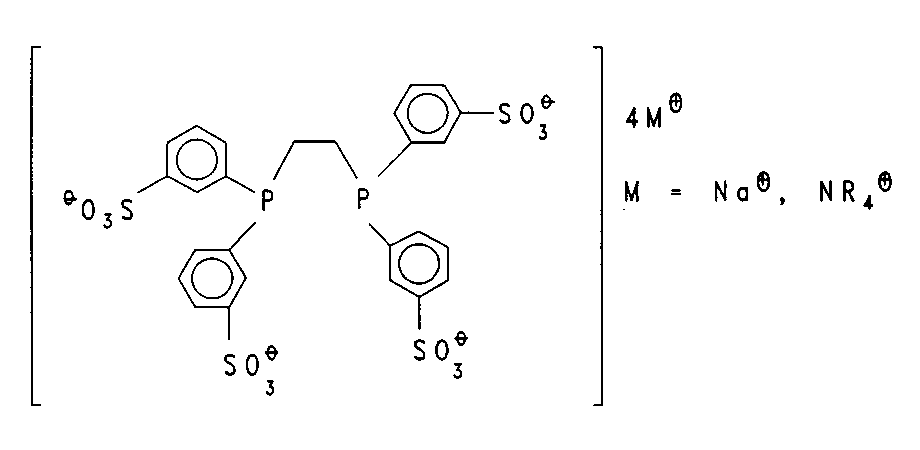 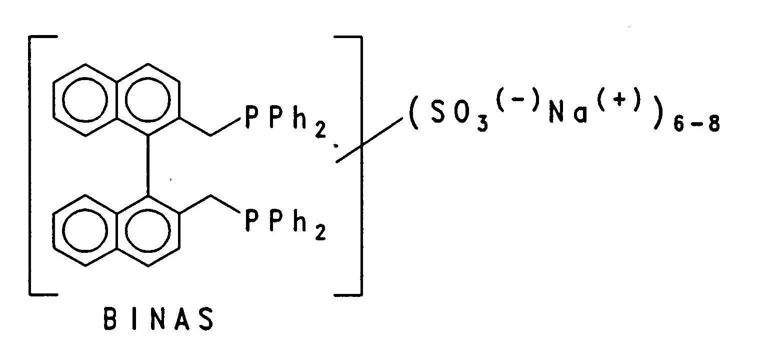 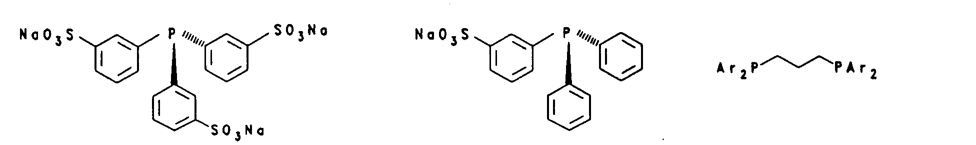 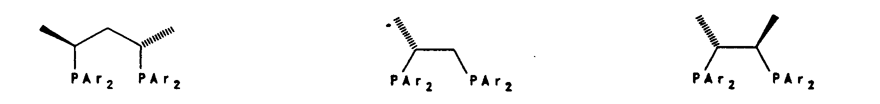 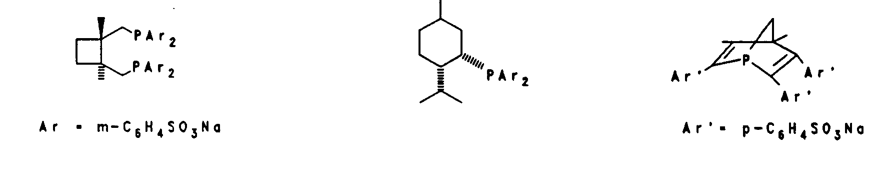 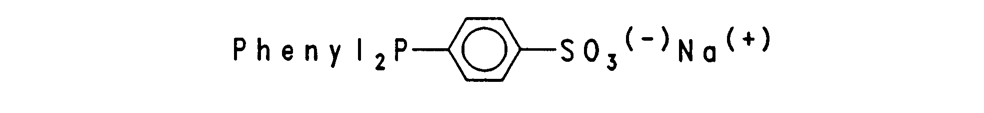 R₃₋ₙP(p-C₆H₄SO₃K)ₙ R = C₆H₅, 2-Pyridyl, 3-Pyridyl; n = 1-3 P[p-OC₆H₄SO₃(NH(i-octyl)₃]₃
1.1 Phosphane mit hydrophilen Gruppen in der Peripherie 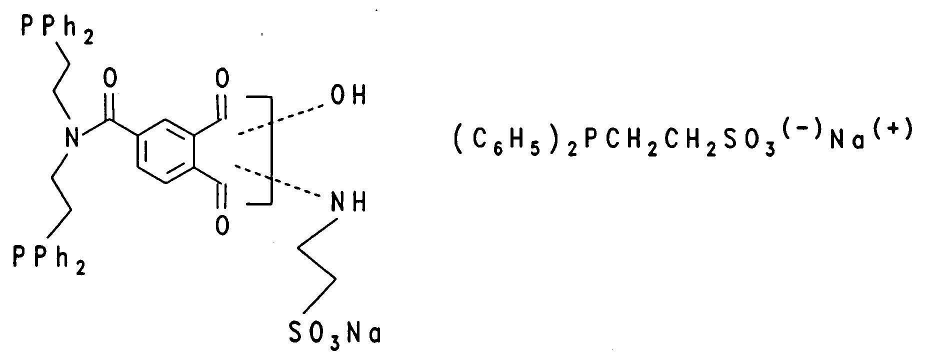 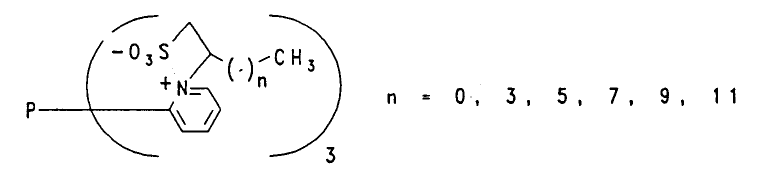
2. Phosphane mit quaternisierten Aminoalkyl- und Aminoaryl-Substituenten 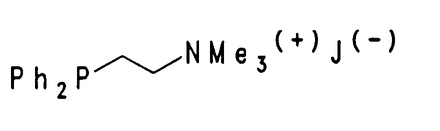 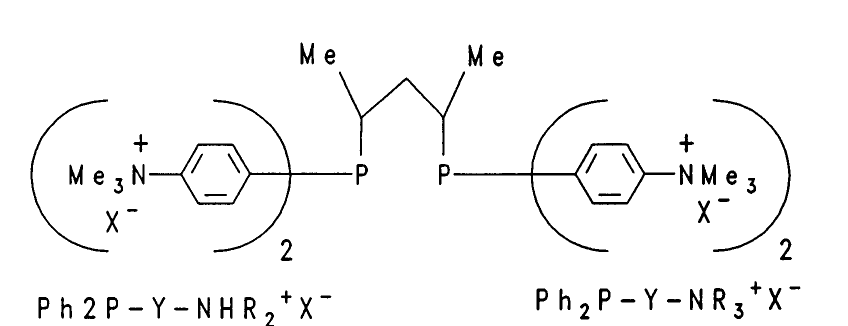 Y = -CH₂CH₂-, -CH(CH₃)CH₂-, -CH₂CH(CH₃)CH₂-; R = CH₃; X = J^{⊖}, Bu^{⊖} , Cl^{⊖}, OSO₂CF₃^{⊖}, BF₄^{⊖}, PF₆^{⊖}
3. Carboxylierte Phosphane 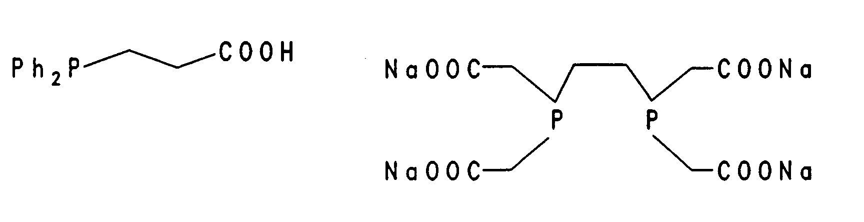 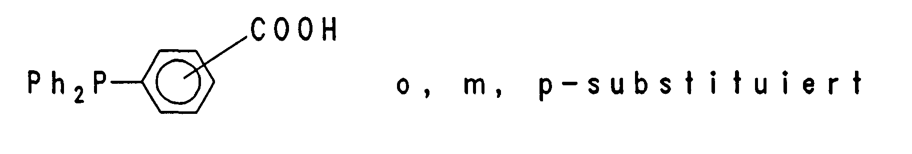 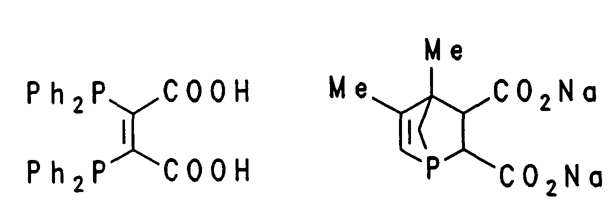
4. Phosphate mit Hydroxyalkyl- oder Polyether-Substituenten 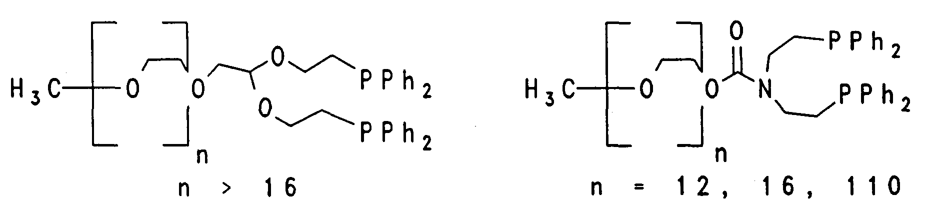 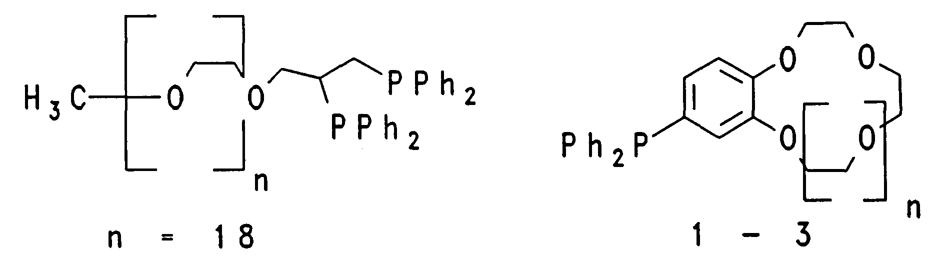
5. Phosphinoalkyl-phosphoniumsalze 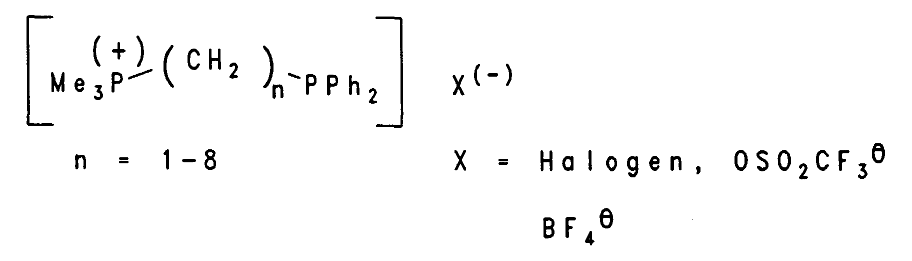
6. Phosphite

   P[-OC₆H₄SO₃{NH(i-Octyl)₃}]₃

Insbesondere bevorzugte wasserlösliche Phosphanliganden sind:

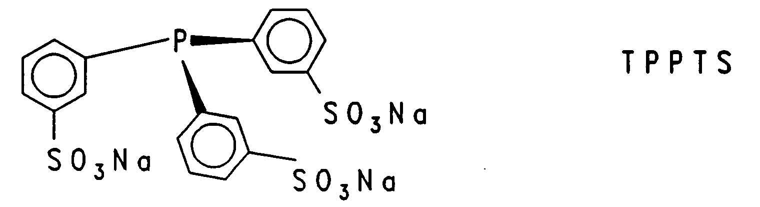

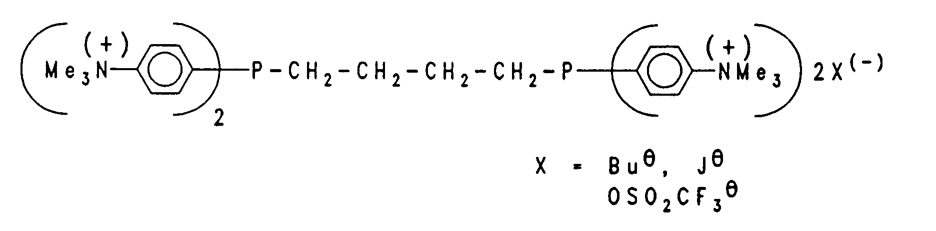

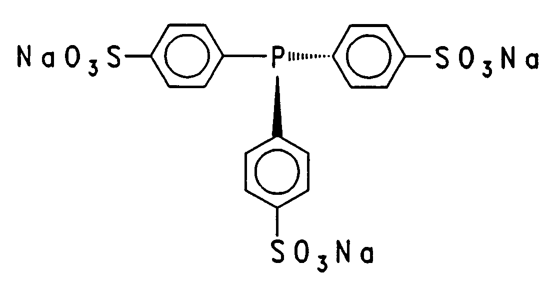

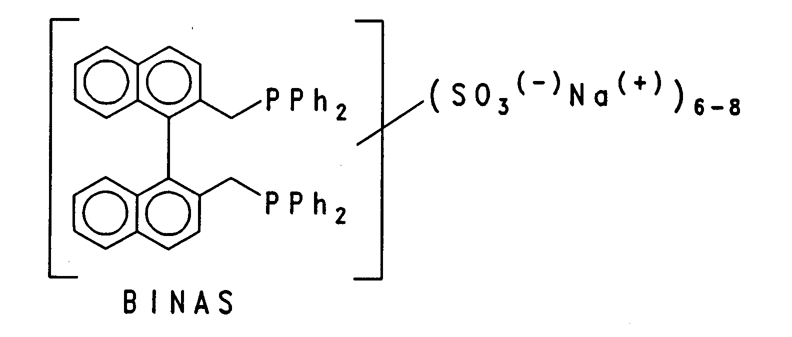

Der wasserlösliche Ligand wird bei dem erfindungsgemäßen Verfahren mit einem Anteil von 0,001 bis 20 Mol-%, bevorzugt 0,01 bis 15 Mol-%, besonders bevorzugt 0,05 bis 10 Mol-%, insbesondere bevorzugt 0,1 bis 6 Mol-%, bezogen auf die aromatische Halogenverbindung oder das Perfluoralkylsulfonat, eingesetzt.
Es können gegebenenfalls auch Mischungen zweier oder mehrerer verschiedener wasserlöslicher Komplexliganden eingesetzt werden.
Die erfindungsgemäß eingesetzten wasserlöslichen Komplexliganden sind zum großen Teil aus der Literatur bekannt. Die Synthesen dieser Verbindungen sind beispielsweise beschrieben in W.A. Herrmann und C.W. Kohlpainter, Angew. Chem. Int. Ed. Engl. 32 (1993) 1524 und der dort zitierten Literatur oder können nach literaturbekannten oder analogen dem Fachmann geläufigen Methoden erfolgen. Die Herstellung von BINAS ist beispielsweise in der deutschen Patentanmeldung P 42 44 274 beschrieben.

Ausgangsverbindungen für das erfindungsgemäße Verfahren sind zum einen aromatische Borverbindungen der Formel (VIII),

Aryl - BQ₁Q₂ (VIII)

worin
- Aryl: ein aromatischer Rest ist und
- Q₁, Q₂: gleich oder verschieden -OH, C₁-C₄-Alkoxy, C₁-C₄-Alkyl, Phenyl, das gegebenenfalls durch C₁-C₄-Alkyl, C₁-C₄-Alkoxy oder Halogen substituiert sein kann, oder Halogen bedeuten oder Q₁ und Q₂ zusammen bilden eine C₁-C₄-Alkylendioxy-Gruppe, eine Methylengruppe, die gegebenenfalls durch eine oder zwei C₁-C₄-Alkylgruppen substituiert sein kann, oder Q₁ und Q₂ und das Boratom zusammen sind Teil eines Boroxinrings der Formel (IX):

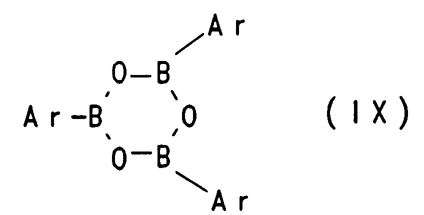

Bevorzugt sind aromatische Borverbindungen der Formel (X),

R¹(-A¹)ₖ(-M¹)ₗ-A²-B Q₁Q₂ (X)

wobei R¹, A¹, A², M¹, Q₁, Q₂, k und l die folgenden Bedeutungen haben:
R¹ ist Benzyloxy, H, F, Cl, Br, -NC, -CN, -CF₃, -OCF₃ oder ein geradkettiger, verzweigter (mit oder ohne asymmetrisches C-Atom) oder cyclischer Alkylrest mit 1 bis 18 C-Atomen, wobei auch eine oder zwei nicht benachbarte -CH₂-Gruppen durch -O-, -S-, -CO-, -CO-O-, -O-CO-, -CO-S-, -S-CO-, -O-CO-O-, -CH=CH-, -C≡C-, -SO₂-, -CON(H, C₁-C₈-Alkyl)-, Cyclopropan-1,2-diyl oder -Si(CH₃)₂- ersetzt sein können, und wobei auch ein oder mehrere H-Atome des Alkylrestes durch F, Cl, Br oder CN substituiert sein können;
A¹ ist 1,4-Phenylen, Pyrazin-2,5-diyl, Pyridazin-3,6-diyl, Pyridin-2,5-diyl, Pyrimidin-2,5-diyl, trans-1,4-Cyclohexylen, Naphthalin-2,6-diyl, wobei ein oder mehrere H Atome gleich oder verschieden durch Substituenten L ersetzt sein können, wobei L die unter R¹ genannten Bedeutungen hat oder 4,4-Dimethylisoxazolin ist, und wobei eine oder zwei nicht benachbarte CH₂-Gruppen des Cyclohexylens durch -O- oder -S- ersetzt sein können, (1,3,4)-Thiadiazol-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Piperidin-1,4-diyl, Bicyclo[2.2.2]octan-1,4-diyl, 1,3-Dioxaborinan-2,5-diyl oder trans-Dekalin-2,6-diyl;
A² ist 1,4-Phenylen, Pyrazin-2,5-diyl, Pyridazin-3,6-diyl, Pyridin-2,5-diyl, Pyrimidin-2,5-diyl, Naphthalin-2,6-diyl, wobei ein oder mehrere H Atome gleich oder verschieden durch Substituenten L ersetzt sein können, wobei L die unter R¹ genannten Bedeutungen hat oder 4,4-Dimethylisoxazolin ist, 1,3,4-Thiadiazol-2,5-diyl, 1,3-Thiazol-2,4-diyl, 1,3-Thiazol-2,5-diyl, Thiophen-2,4-diyl oder Thiophen-2,5-diyl;
M¹ ist -O-, -S-, -CO-, -CO-O-, O-CO-, -CO-S, -S-CO-, -O-CO-O-, -CH₂-O-, -OCH₂-, -CH₂CH₂-, -CH=CH-, -C≡C-, -CH(CN)-CH₂-, -CH₂-CH(CN)-, -CH=N-, -N=CH-, -CH₂CH₂CH₂-O-, -OCH₂CH₂CH₂-, -CH₂CH₂CO-O-, -O-COCH₂CH₂-, und
   - Q₁, Q₂: sind gleich oder verschieden -OH, C₁-C₄-Alkoxy oder Halogen oder Q₁ und Q₂ zusammen bilden eine C₁-C₄-Alkylendioxy-Gruppe oder Q₁ und Q₂ und das Boratom zusammen sind teil eines Boroxinrings der Formel (IX): 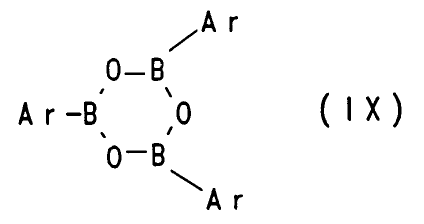
k, l bedeuten jeweils unabhängig voneinander Null oder Eins.

Bevorzugt bedeutet R¹, Benzyloxy, H, F, Cl, -CF₃, OCF₃, CN oder einen geradkettigen, verzweigten (mit oder ohne asymmetrisches C-Atom) oder cyclischen Alkylrest mit 1 bis 18 C-Atomen, wobei auch eine oder zwei nicht benachbarte -CH₂-Gruppen durch -O-, -CO-, -CO-O-, -OCO-, -O-CO-O-, -CH=CH-, -C≡C-, Cyclopropan-1,2-diyl oder -Si(CH₃)₂- ersetzt sein können, und wobei auch ein oder mehrere H-Atome des Alkylrestes durch F, Cl oder CN substituiert sein können.

Besonders bevorzugt bedeutet R¹ Benzyloxy, H oder einen geradkettigen, verzweigten (mit oder ohne asymmetrisches C-Atom) oder cyclischen Alkylrest mit 1 bis 18 C-Atomen, wobei auch eine oder zwei nicht benachbarte -CH₂-Gruppen durch -O-, -CH=CH-, -C≡C-, Cyclopropan-1,2-diyl oder -Si(CH₃)₂- ersetzt sein können.

Bevorzugt bedeutet A¹ 1,4-Phenylen, Pyrazin-2,5-diyl, Pyridazin-3,6-diyl, Naphthalin-2,6-diyl, Pyridin-2,5-diyl, Pyrimidin-2,5-diyl, wobei ein oder mehrere H-Atome gleich oder verschieden durch Substituenten L ersetzt sein können, wobei L die unter R¹ genannten Bedeutungen hat, trans-1,4-Cyclohexylen, worin eine oder zwei nicht benachbarte -CH₂-Gruppen durch -O- ersetzt sein können, (1,3,4)-Thiadiazol-2,5-diyl, oder Bicyclo[2.2.2]octan-1,4-diyl.

Besonders bevorzugt bedeutet A¹ 1,4-Phenylen, 2-Fluor-1,4-Phenylen, 2,3-Difluor-1,4-Phenylen, 2,6-Difluor-1,4-Phenylen, 2,5-Difluor-1,4-Phenylen, sowie Pyridin-2,5-diyl, Pyrimidin-2,5-diyl, wobei ein oder zwei H-Atome gleich oder verschieden durch Substituenten L ersetzt sein können, wobei L die unter R¹ genannten Bedeutungen hat, trans-1,4-Cyclohexylen.

Bevorzugt bedeutet A² 1,4-Phenylen, Pyrazin-2,5-diyl, Pyridazin-3,6-diyl, Pyridin-2,5-diyl, Pyrimidin-2,5-diyl, Naphthalin-2,6-diyl, wobei ein oder mehrere H-Atome gleich oder verschieden durch Substituenten L ersetzt sein können, wobei L die unter R¹ genannten Bedeutungen hat.

Besonders bevorzugt bedeutet A² 1,4-Phenylen, 2-Fluor-1,4-Phenylen, 2,3-Difluor-1,4-phenylen, 2,6-Difluor-1,4-Phenylen, 2,5-Difluor-1,4-phenylen, sowie Pyridin-2,5-diyl, oder Naphthalin-2,6-diyl, wobei ein oder mehrere H-Atome gleich oder verschieden durch Substituenten L ersetzt sein können, wobei L die unter R¹ genannten Bedeutungen hat.

Bevorzugt bedeutet M¹ -O-, -CO-, -CO-O-, -O-CO-, -O-CO-O-, -CH₂-O-, -O-CH₂-, -CH₂-CH₂-, -CH=CH-, -C≡C-, -CH₂CH₂CH₂-O-, -O-CH₂CH₂CH₂-, -CH₂CH₂CO-O- oder -O-CO-CH₂CH₂-.

Besonders bevorzugt bedeutet M¹ -O-, -CH₂-O-, -O-CH₂-, -CH₂CH₂-, -CH=CH- oder -C≡C-.

Insbesondere bevorzugt sind nachfolgend aufgeführte aromatische Boronsäuren der Formel (Xa) bis (Xh): 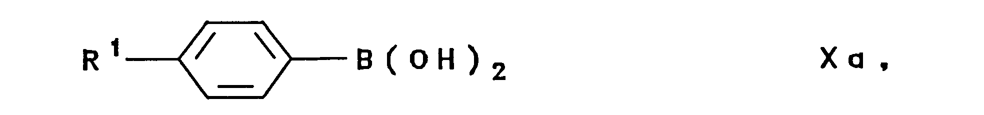 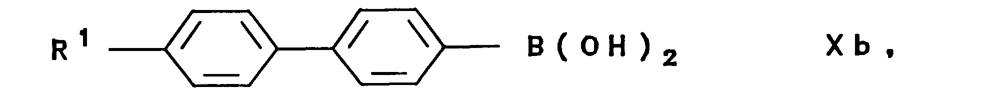 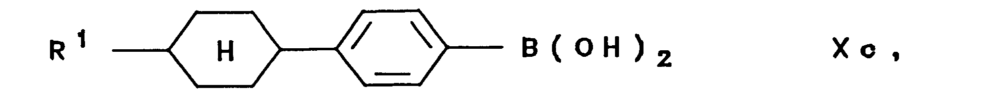 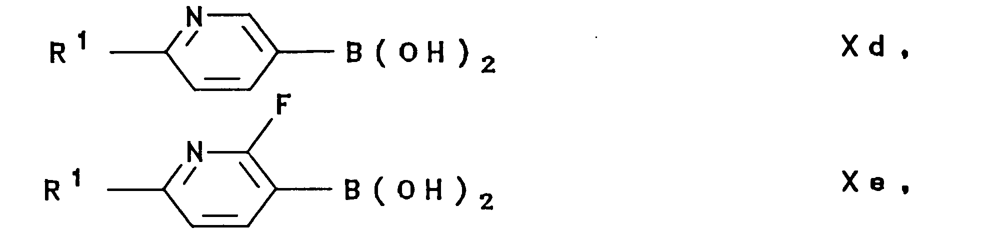 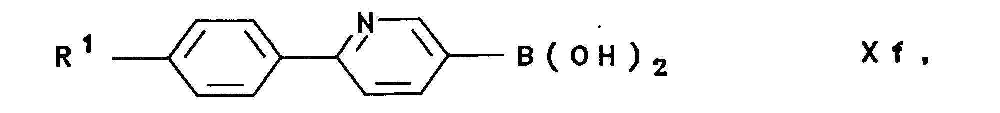 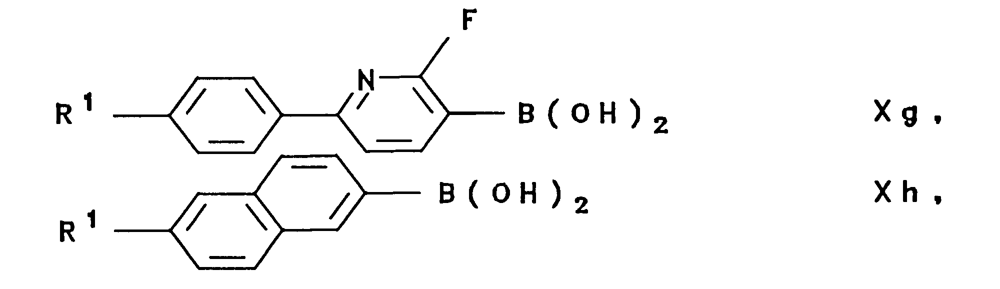 wobei R¹ Benzyloxy, H, Methyl, Ethyl, Propyl, Butyl, Pentyl, Hexyl, Heptyl, Octyl, Nonyl, Decyl, Undecyl, Dodecyl, Tridecyl, Tetradecyl und Pentadecyl, sowie Methoxy, Ethoxy, Propoxy, Butoxy, Pentoxy, Hexoxy, Heptoxy, Octoxy, Nonoxy, Decoxy, Undecoxy, Dodecoxy, Tridecoxy, Tetradecoxy oder Pentadecoxy bedeutet.

Die verwendeten aromatischen Borverbindungen sind entweder bekannt oder können nach bekannten Methoden, wie beispielsweise in Houben Weyl Methoden der Organischen Chemie, Georg Thieme-Verlag, Stuttgart, Band 13/3a beschrieben, hergestellt werden. So ist es beispielsweise möglich, aus aromatischen Alkalimetall- und Magnesiumverbindungen durch Umsetzung mit Trialkoxyboranen und anschließender Hydrolyse Boronsäuren, vorzugsweise solche der Formel (II), zu erhalten.

Die zweite Klasse von Ausgangsverbindungen für das erfindungsgemäße Verfahren sind aromatische Halogenverbindungen oder aromatische Perfluoralkylsulfonate, vorzugsweise solche der Formel (XI),

X-A³(-M²)ₘ(-A⁴)ₙ-R² (XI),

wobei R², A³, A⁴, M², X m und n die folgenden Bedeutungen haben:
R² ist Benzyloxy, H, F, Cl, Br, -NC, -CN, -CF₃, -OCF₃, Isoxazolin oder ein geradkettiger, verzweigter (mit oder ohne asymmetrisches C-Atom) oder cyclischer Alkylrest mit 1 bis 18 C-Atomen, wobei auch eine oder zwei nicht benachbarte -CH₂-Gruppen durch -O-, -S-, -CO-, -CO-O-, -O-CO-, -CO-S-, -S-CO-, -O-CO-O-, -SO₂-, -CON(H,C₁-C₈-Alkyl)-, -CH=CH-, -C≡C-, Cyclopropan-1,2-diyl oder -Si(CH₃)₂- ersetzt sein können, und wobei auch ein oder mehrere H-Atome des Alkylrestes durch F, Cl, Br oder CN substituiert sein können,
A⁴ ist 1,4-Phenylen, Pyrazin-2,5-diyl, Pyridazin-3,6-diyl, Pyridin-2,5-diyl, Pyrimidin-2,5-diyl, trans-1,4-Cyclohexylen, Naphthalin-2,6-diyl, wobei ein oder mehrere H-Atome gleich oder verschieden durch Substituenten L ersetzt sein können, wobei L die unter R¹ genannten Bedeutungen hat, -CHO oder 4,4-Dimethylisoxazolin ist, und wobei eine oder zwei nicht benachbarte CH₂-Gruppen des Cyclohexylens durch -O- oder -S- ersetzt sein können, (1,3,4)-Thiadiazol-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Piperidin-1,4-diyl, Bicyclo[2.2.2]octan-1,4-diyl, 1,3-Dioxaborinan-2,5-diyl oder trans-Dekalin-2,6-diyl;
A³ ist 1,4-Phenylen, Pyrazin-2,5-diyl, Pyridazin-3,6-diyl, Pyridin-2,5-diyl, Pyrimidin-2,5-diyl, Naphthalin-2,6-diyl, wobei ein oder mehrere H-Atome gleich oder verschieden durch Substituenten L ersetzt sein können, wobei L die unter R¹ genannten Bedeutungen hat, -CHO oder 4,4-Dimethylisoxazolin ist, 1,3,4-Thiadiazol-2,5-diyl, 1,3-Thiazol-2,4-diyl, 1,3-Thiazol-2,5-diyl, Thiophen-2,4-diyl oder Thiophen-2,5-diyl;
M² ist -O-, -S-, -CO-, -CO-O-, O-CO-, -CO-S, -S-CO-, -O-CO-O-, -CH₂-O-, -OCH₂-, -CH₂CH₂-, -CH=CH-, -C≡C-, -CH(CN)-CH₂-, -CH₂-CH(CN)-, -CH=N-, -N=CH-, -CH₂CH₂CH₂-O-, -OCH₂CH₂CH₂-, -CH₂CH₂CO-O-, -O-COCH₂CH₂-;
X ist Cl, Br, I oder Perfluoralkylsulfonat; und
m, n bedeuten jeweils unabhängig voneinander Null oder Eins.

Bevorzugt bedeutet R² Benzyloxy, H, F, Cl, Br, -CN, -CF₃, -OCF₃ oder einen geradkettigen, verzweigten (mit oder ohne asymmetrisches C-Atom) oder cyclischen Alkylrest mit 1 bis 18 C-Atomen, wobei auch eine oder zwei nicht benachbarte -CH₂-Gruppen durch -O-, -CO-, -CO-O-, -O-CO-, -O-CO-O-, -CH=CH-, -C≡C-, Cyclopropan-1,2-diyl oder -Si(CH₃)₂- ersetzt sein können, und wobei auch ein oder mehrere H-Atome des Alkylrestes durch F, Cl oder CN substituiert sein können.

Besonders bevorzugt bedeutet R² Benzyloxy, H, Cl, Br oder einen geradkettigen, verzweigten (mit oder ohne assymmetrisches C-Atom) oder cyclischen Alkylrest mit 1 bis 18 C-Atomen, wobei auch eine oder zwei nicht benachtbarte -CH₂-Gruppen durch -O-, -CO-, -CO-O-, -O-CO-, -O-CO-O-, -CH=CH-, -C≡C-, Cyclopropan-1,2-diyl oder -Si(CH₃)-ersetzt sein können.

Bevorzugt bedeutet A³ 1,4-Phenylen, Pyrazin-2,5-diyl, Pyridazin-3,6-diyl, Pyridin-2,5-diyl, Pyrimidin-2,5-diyl, Naphthalin-2,6-diyl, wobei ein oder mehrere H-Atome gleich oder verschieden durch Substituenten L ersetzt sein können, wobei L die unter R¹ genannten Bedeutungen hat, -CHO oder 4,4-Dimethylisoxazolin ist, oder 1,3,4-Thiadiazol-2,5-diyl.

Besonders bevorzugt bedeutet A³ 1,4-Phenylen, 2-Fluor-1,4-phenylen, 2,3-Difluor-1,4-phenylen, 2,6-Difluor-1,4-phenylen, 2,5-Difluor-1,4-phenylen, sowie Pyrazin-2,5-diyl, Pyridazin-2,5-diyl, Pyrimidin-2,5-diyl oder Naphthalin-2,6-diyl, wobei ein oder mehrere H-Atome gleich oder verschieden durch Substituenten L ersetzt sein können, wobei L die unter R¹ genannten Bedeutungen hat, -CHO oder 4,4-Dimethylisoxazolin ist.

Bevorzugt bedeutet A⁴ 1,4-Phenylen, Pyrazin-2,5-diyl, Pyridazin-3,6-diyl, Pyridin-2,5-diyl, Pyrimidin-2,5-diyl, trans-1,4-Cyclohexylen, Naphthalin-2,6-diyl, wobei ein oder mehrere H-Atome gleich oder verschieden durch Substituenten L ersetzt sein können, wobei L die unter R¹ genannten Bedeutungen hat, und wobei eine oder zwei nicht benachbarte CH₂-Gruppen des Cyclohexylens durch -O- ersetzt sein können, (1,3,4)-Thiadiazol-2,5-diyl oder Bicyclo[2.2.2]octan-1,4-diyl.

Besonders bevorzugt bedeutet A⁴ 1,4-Phenylen, 2-Fluor-1,4-phenylen, 2,3-Difluor-1,4-phenylen, 2,6-Difluor-1,4-phenylen, 2,5-Difluor-1,4-phenylen, sowie Pyridin-2,5-diyl, Pyrimidin-2,5-diyl, trans-1,4-Cyclohexylen, Naphthalin-2,6-diyl, wobei ein oder mehrere H-Atome gleich oder verschieden durch Substituenten L ersetzt sein können, wobei L die unter R¹ genannten Bedeutungen hat.

Bevorzugt bedeutet M² -O-, -CO-, -CO-O-, -O-CO-, -O-CO-O-, -CH₂-O-, -O-CH₂-, -CH₂CH₂-, -CH=CH-, -C≡C-, -CH₂CH₂CH₂-O-, -O-CH₂CH₂CH₂-, -CH₂CH₂CO-O- oder -O-CO-CH₂CH₂-.

Besonders bevorzugt bedeutet M² -O-, -CO-, -CO-O-, -O-CO-, -O-CO-O-, -CH₂-O-, -O-CH₂-, -CH₂CH₂-, -CH=CH-, -C≡C-, -CH₂CH₂CH₂-O-, -O-CH₂CH₂CH₂-, -CH₂CH₂CO-O- oder -O-CO-CH₂CH₂.

Bevorzugt bedeutet X Brom, lod oder OSO₂-CₚF_{2p + 1}, worin p einen ganzzahligen Wert von 1 bis 10 darstellt. Besonders bevorzugt bedeutet X Brom.

Insbesondere bevorzugt sind die nachfolgend aufgeführten aromatischen Halogenverbindungen der Formel (XI 1) bis (Xl 31) 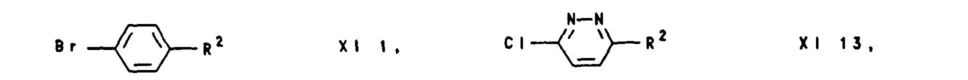 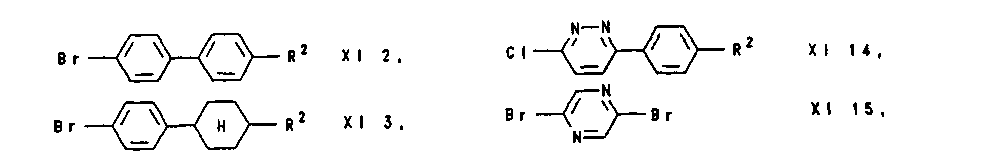 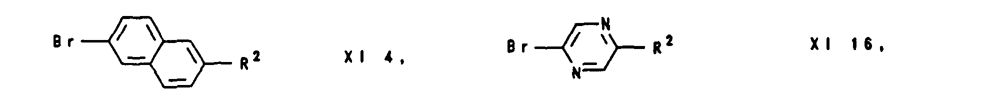 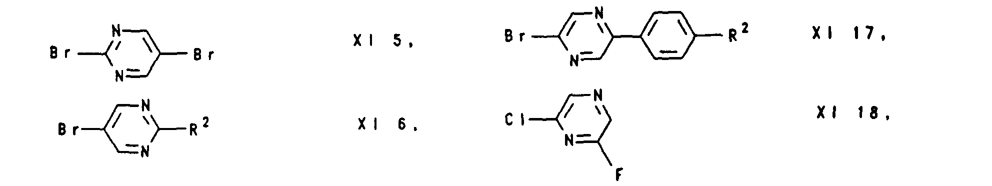 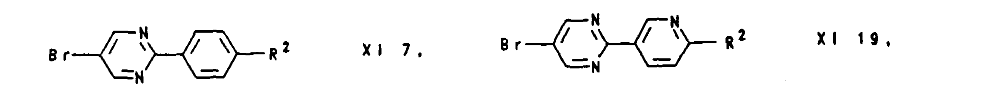 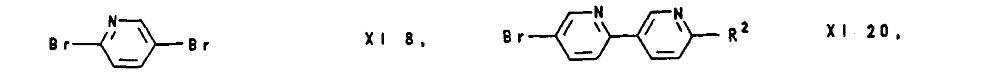 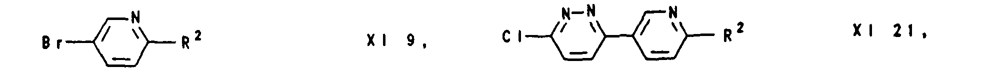 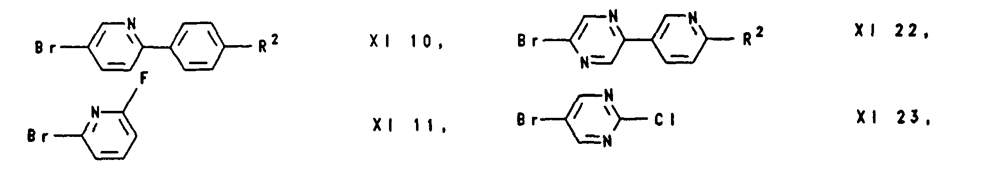 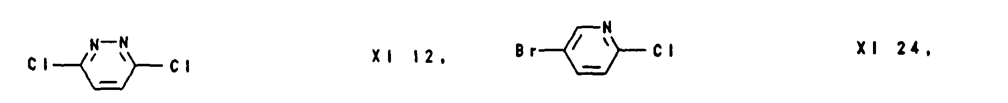 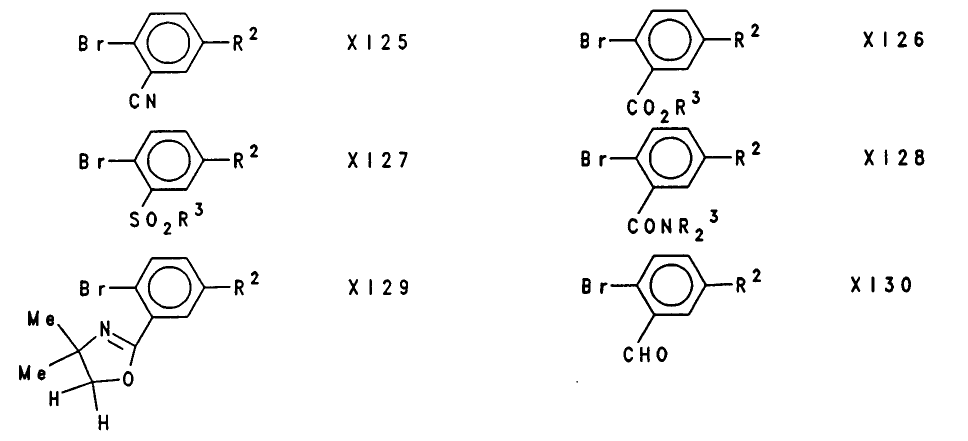 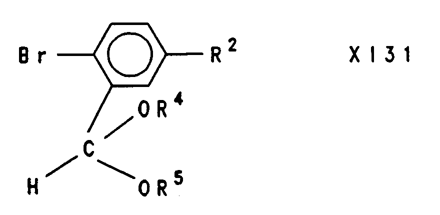 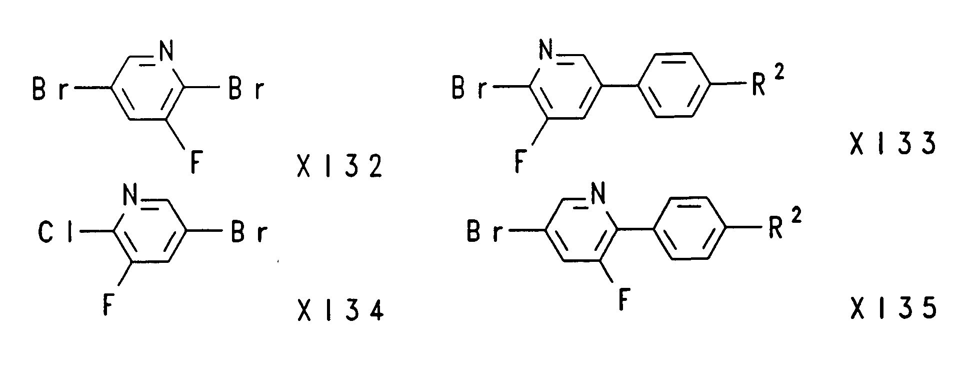 wobei R², R³ Benzyloxy, H, Methyl, Ethyl, Propyl, Butyl, Pentyl, Hexyl, Heptyl, Octyl, Nonyl, Decyl, Undecyl, Dodecyl, Tridecyl, Tetradecyl und Pentadecyl, sowie Methoxy, Ethoxy, Propoxy, Butoxy, Pentoxy, Hexoxy, Heptoxy, Octoxy, Nonoxy, Decoxy, Undecoxy, Dodecoxy, Tridecoxy, Tetradecoxy und Pentadecoxy und R⁴, R⁵ Methyl, Ethyl, Propyl, Butyl, Pentyl, Hexyl, Heptyl, Octyl, Nonyl, Decyl, Undecyl, Tridecyl, Tetradecyl und Pentadecyl oder R⁴ und R⁵ zusammen auch -(CH₂)₂- oder -(CH₂)₃- bedeuten.

Die verwendeten aromatischen Halogenverbindungen und Perfluoralkylsulfonate sind entweder bekannt oder können nach bekannten Methoden, wie beispielsweise in Houben Weyl, Methoden der Organischen Chemie, Georg Thieme Verlag, Stuttgart, Band 5/3 und 5/4 beschrieben, hergestellt werden. Beispielsweise lassen sich aromatische Halogenide dadurch erhalten, daß man in einem entsprechenden Diazoniumsalz die Diazoniumgruppe durch Chlor, Brom oder lod ersetzt.
Des weiteren lassen sich Hydroxy-Stickstoffheterocyclen mit Hilfe von Phosphortrihalogeniden und Phosphoroxytrihalogeniden in die entsprechenden Halogenide überführen.
Das erfindungsgemäße Verfahren zur Kreuzkupplung von aromatischen Borverbindungen mit aromatischen Halogenverbindungen oder Perfluoralkylsulfonaten kann ebenfalls zur Herstellung von Verbindungen der Formel (XI) benutzt werden. Perfluoralkylsulfonate der Formel (III), worin X OSO₂-CₙH_{2n + 1} bedeutet, können durch Veresterung entsprechender Alkohole der Formel (III), worin X eine Hydroxylgruppe bedeutet, mit Perfluoralkansulfonsäuren oder ihren reaktiven Derivaten hergestellt werden. Die entsprechenden Perfluoralkansulfonsäuren sind bekannt. Als reaktionsfähige Derivate der genannten Perfluoralkansulfonsäuren eignen sich insbesondere die Säurehalogenide, vor allem die Chloride und Bromide, ferner die Anhydride.

Produkte des erfindungsgemäßen Verfahrens sind mehrkernige aromatische Verbindungen.

Als bevorzugte Produkte des erfindungsgemäßen Verfahrens fallen Verbindungen der allgemeinen Formel (XII) an,

R¹(-A¹)ₖ(-M¹)ₗ-A²-A³(-M²)ₘ(-A⁴)ₙ-R² (XII)

wobei
R¹ und R² unabhängig voneinander Benzyloxy, H, F, Cl, Br, -NC, -CN, -CF₃, -OCF₃ oder ein geradkettiger, verzweigter (mit oder ohne asymmetrisches C-Atom) oder cyclischer Alkylrest mit 1 bis 18 C-Atomen sein können, wobei auch eine oder zwei nicht benachbarte -CH₂-Gruppen durch -O-, -S-, -CO-, -CO-O-, -O-CO-, -CO-S-, -S-CO-, -O-CO-O-, -CH=CH-, -C≡C-, -SO₂-, CON(H,C₁-C₈-Alkyl), Cyclopropan-1,2-diyl oder -Si(CH₃)₂- ersetzt sein können, und wobei auch ein oder mehrere H-Atome des Alkylrestes durch F, Cl, Br oder CN substituiert sein können;
A¹ und A⁴ jeweils unabhängig voneinander 1,4-Phenylen, Pyrazin-2,5-diyl, Pyridazin-3,6-diyl, Pyridin-2,5-diyl, Pyrimidin-2,5-diyl, trans-1,4-Cyclohexylen, Naphthalin-2,6-diyl, wobei ein oder mehrere H-Atome gleich oder verschieden durch Substituenten L ersetzt sein können, wobei L die unter R¹ genannten Bedeutungen hat oder 4,4-Dimethylisoxazolin ist, und wobei eine oder zwei nicht benachbarte CH₂-Gruppen des Cyclohexylens durch -O- oder -S- ersetzt sein können, (1,3,4)-Thiadiazol-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Piperidin-1,4-diyl, Bicyclo[2.2.2]octan-1,4-diyl, 1,3-Dioxaborinan-2,5-diyl oder trans-Dekalin-2,6-diyl;
A² und A³ jeweils unabhänig voneinander 1,4-Phenylen, Pyrazin-2,5-diyl, Pyridazin-3,6-diyl, Pyridin-2,5-diyl, Pyrimidin-2,5-diyl, Naphthalin-2,6-diyl, wobei ein oder mehrere H-Atome gleich oder verschieden durch Substituenten L ersetzt sein können, wobei L die unter R¹ genannten Bedeutungen hat, 4,4-Dimethylisoxazolin oder im Falle von A³ auch -CHO ist, 1,3,4-Thiadiazol-2,5-diyl, 1,3-Thiazol-2,4-diyl, 1,3-Thiazol-2,5-diyl, Thiophen-2,4-diyl oder Thiophen-2,5-diyl sein können;
M¹ und M² jeweils unabhänig voneinander -O-, -S-, -CO-, -CO-O-, O-CO-, -CO-S, -S-CO-, -O-CO-O-, -CH₂-O-, -OCH₂-, -CH₂CH₂-, -CH=CH-, -C≡C-, -CH(CN)-CH₂-, -CH₂-CH(CN)-, -CH=N-, -N=CH-, -CH₂CH₂CH₂-O-, -OCH₂CH₂CH₂-, -CH₂CH₂CO-O-, -O-COCH₂CH₂- sein können; und
k, l, m, n jeweils unabhängig voneinander Null oder Eins bedeuten.

Bevorzugte und besonders bevorzugte Varianten von R¹, R², A¹, A², A³, A⁴, M¹, M², k, l, m, n sind die in den Formeln (II) und (III) angegebenen.

Insbesondere bevorzugt sind die nachfolgend aufgeführten Verbindungen der Formel (XII 1) bis (XII 101) 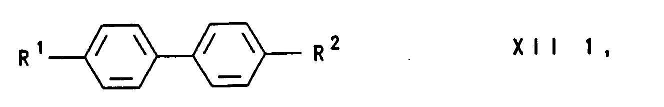 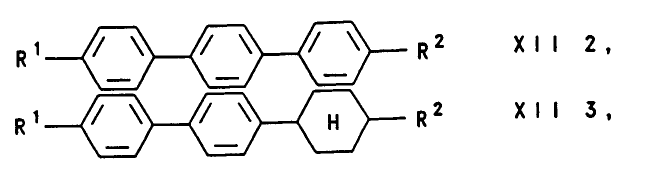 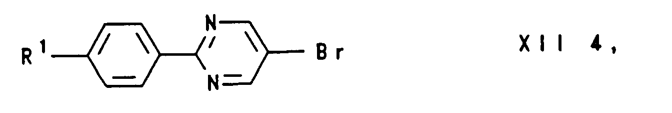 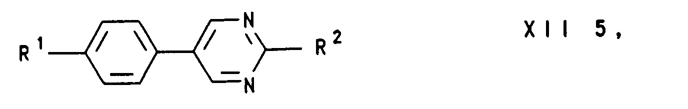 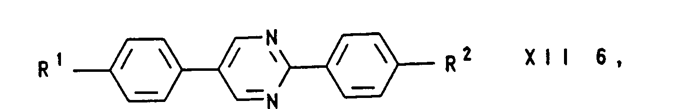 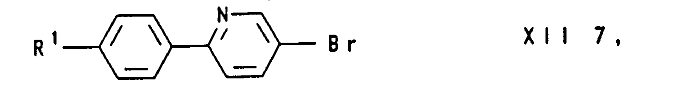 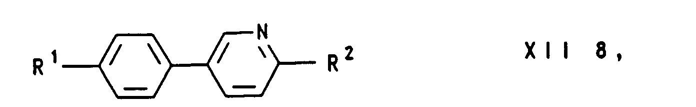 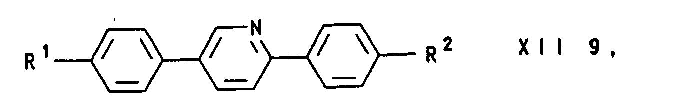 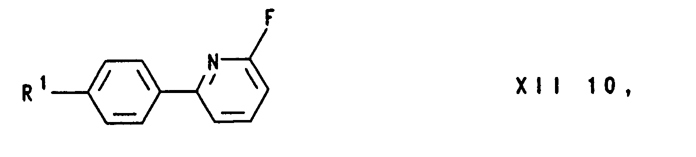 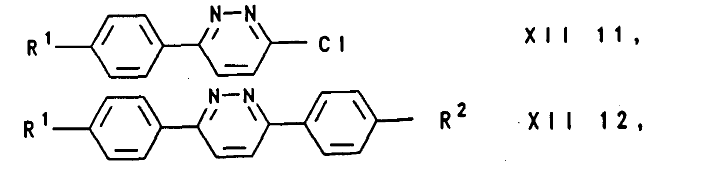 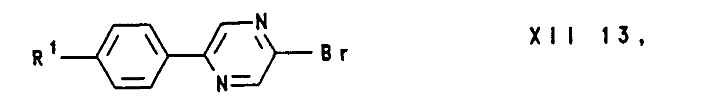 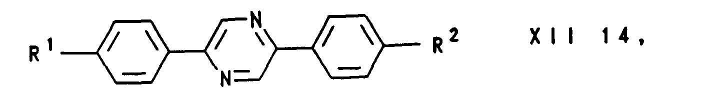 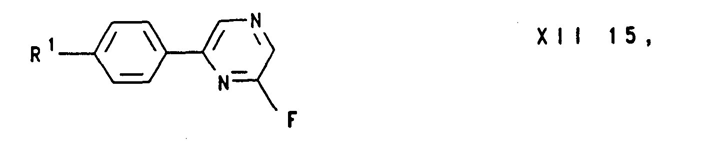 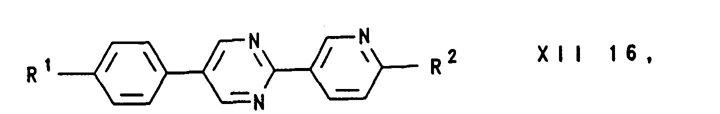 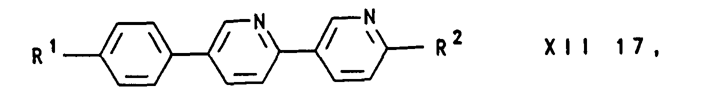 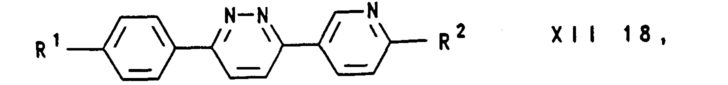 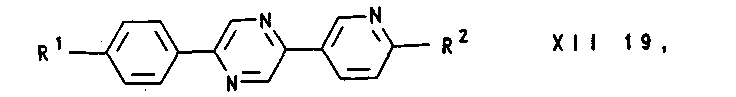 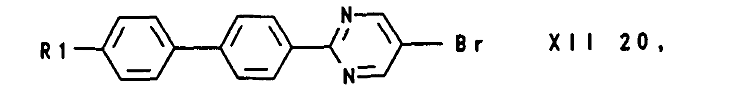 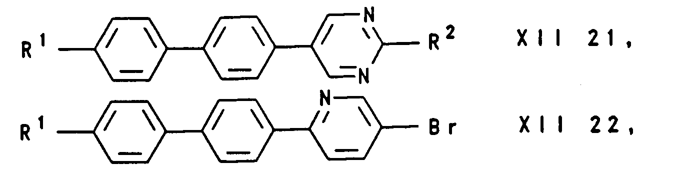 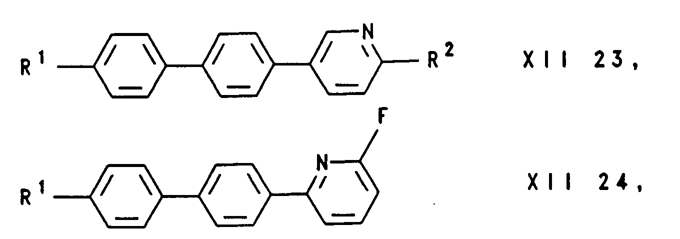 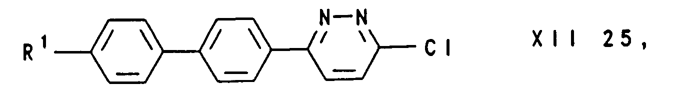 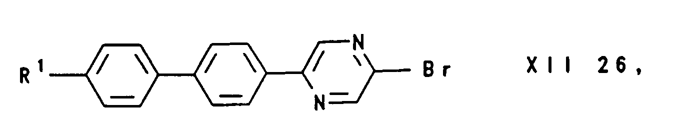 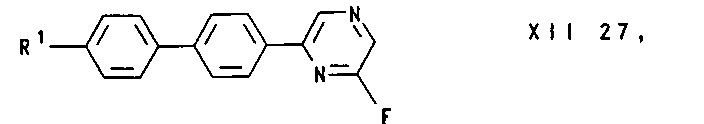 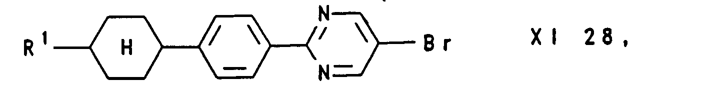 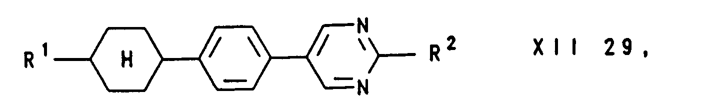 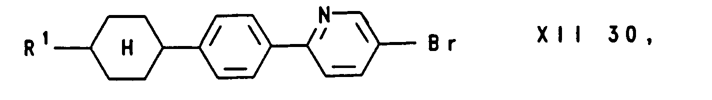 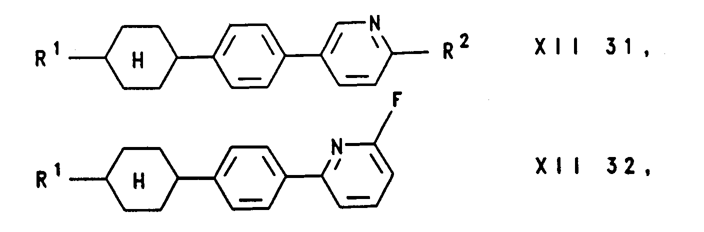 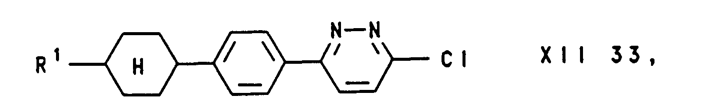 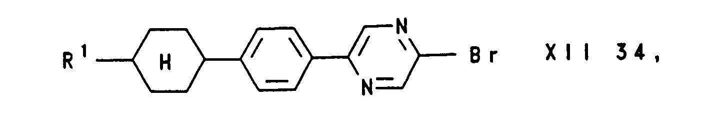 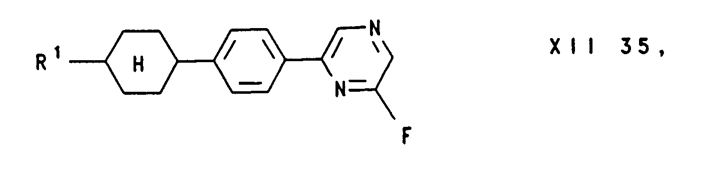 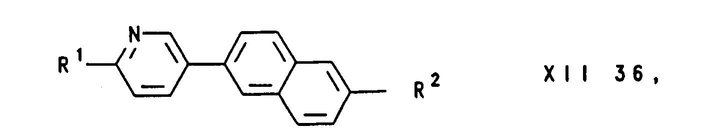 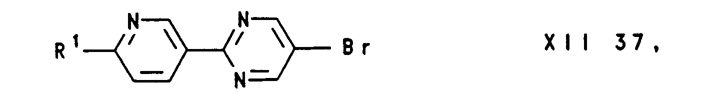 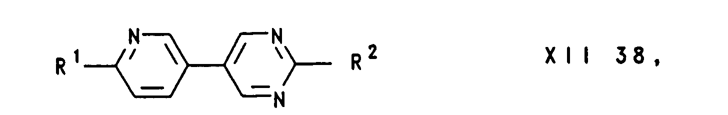 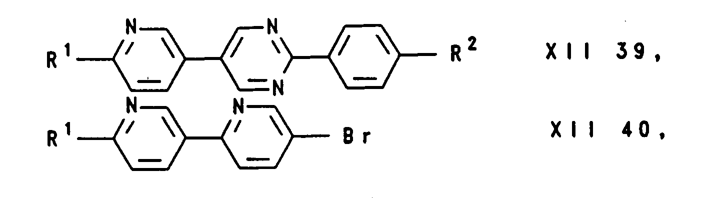 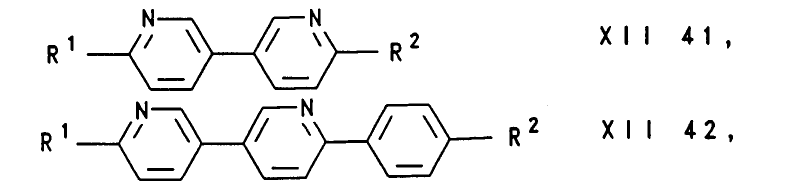 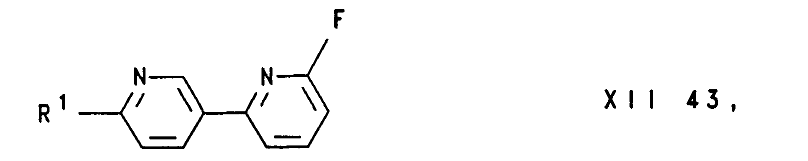 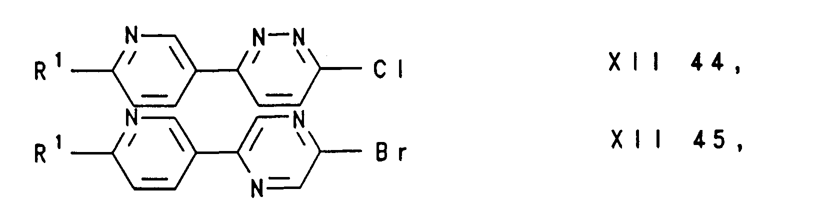 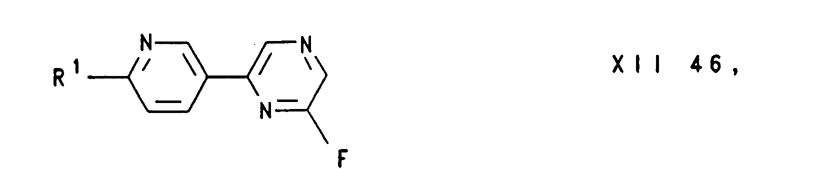 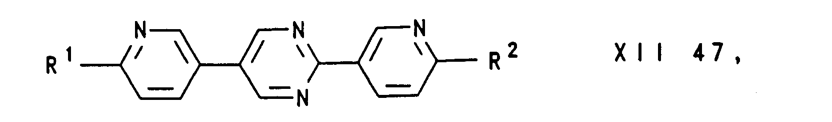 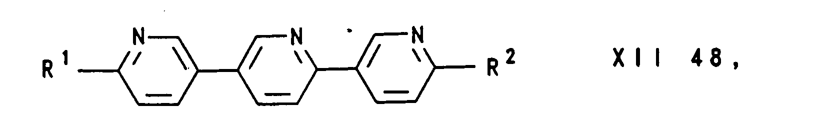 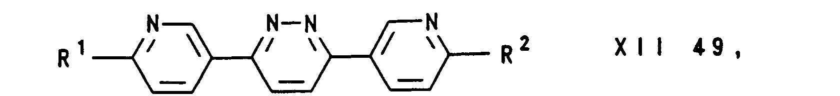 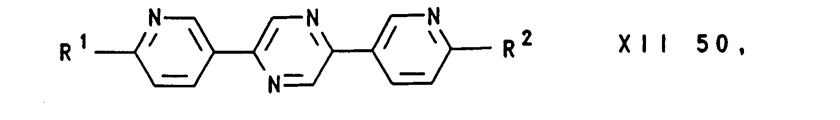 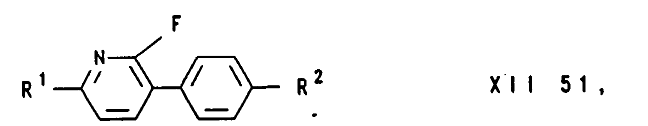 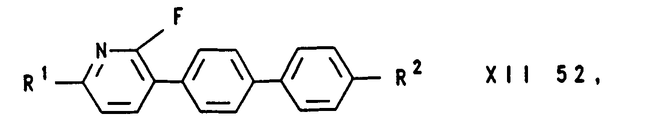 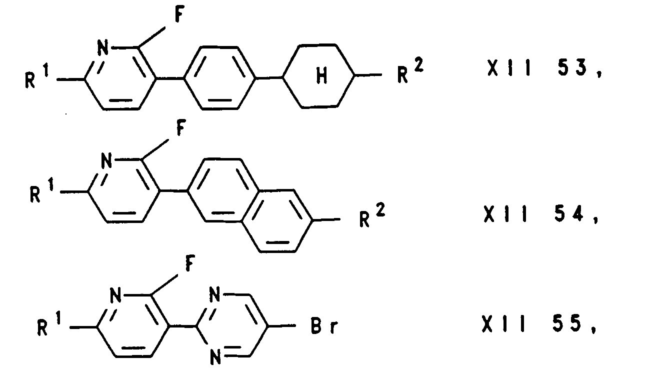 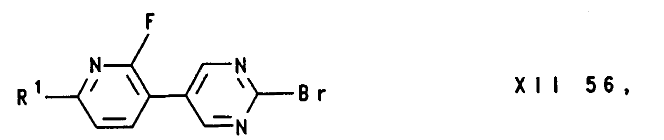 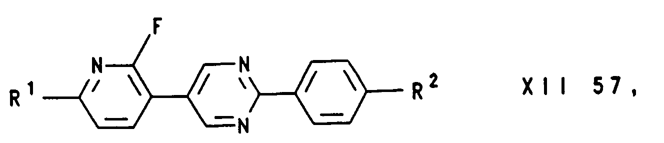 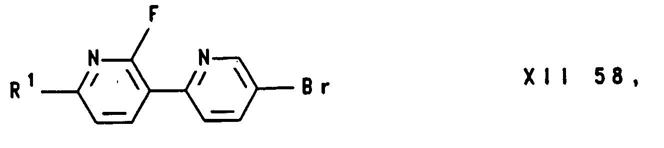 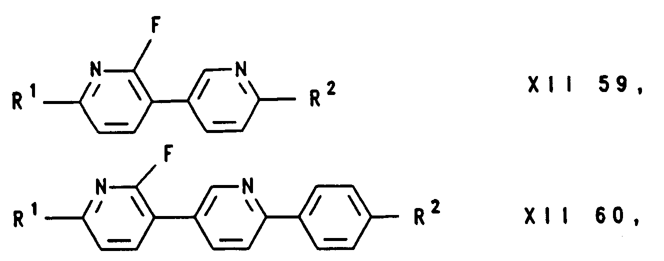 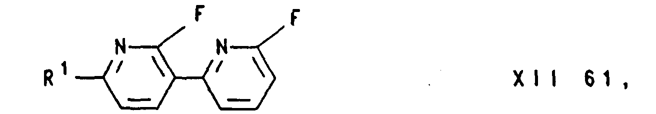 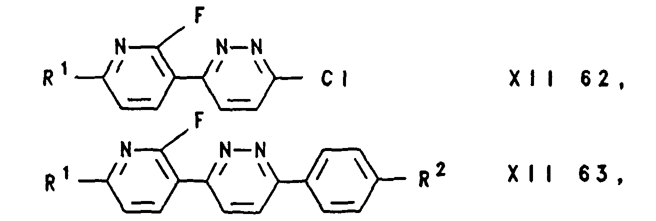 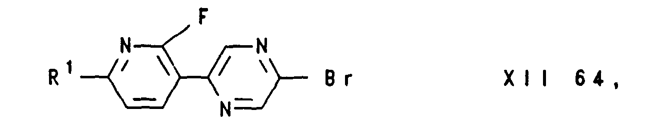 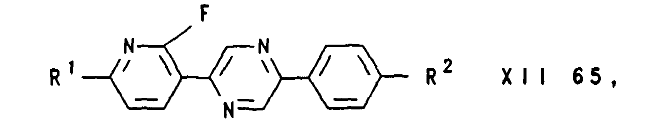 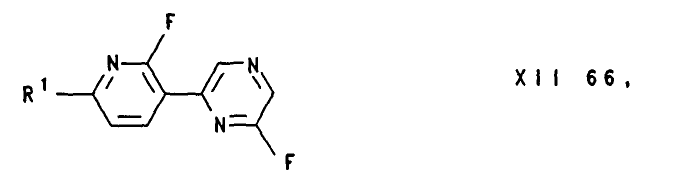 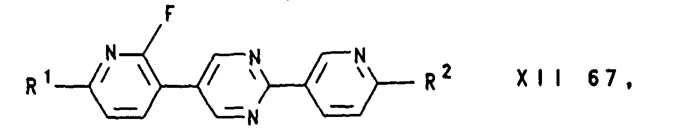 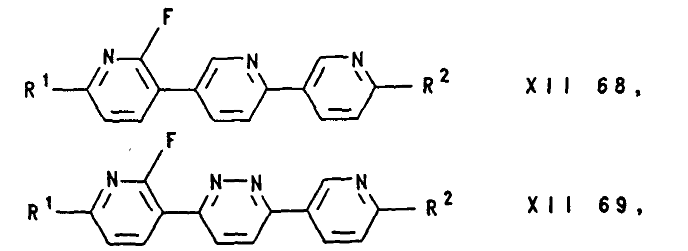 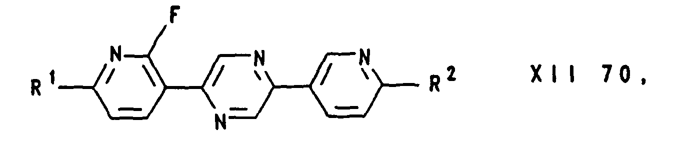 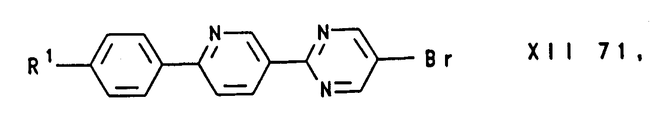 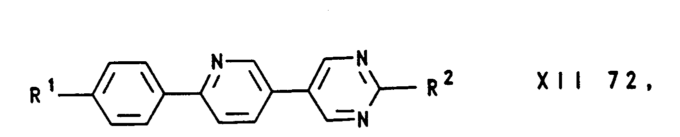 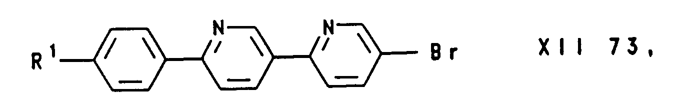 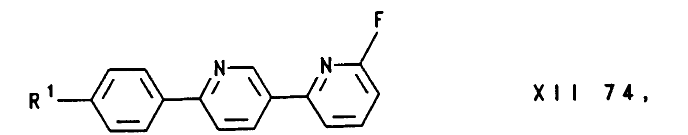 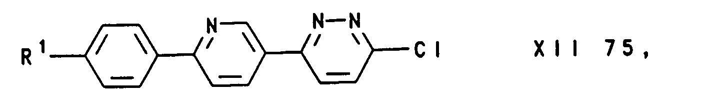 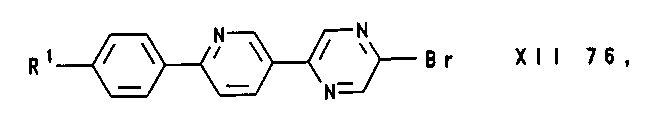 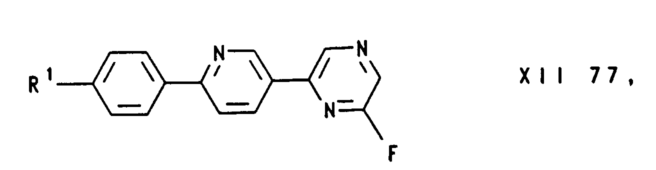 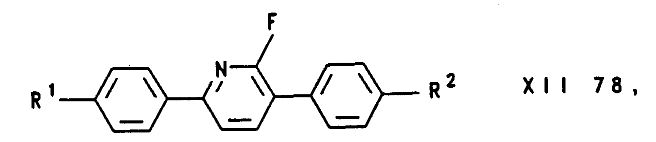 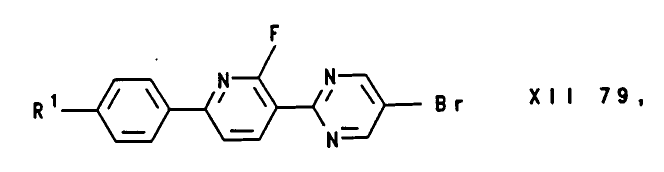 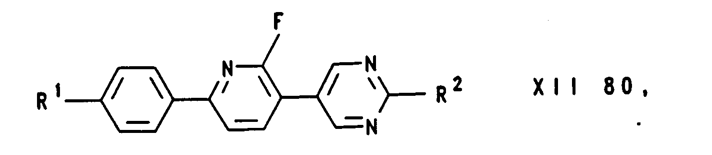 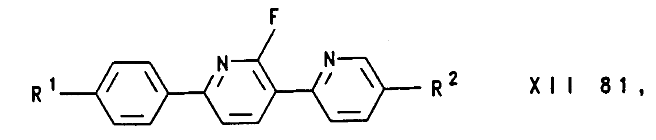 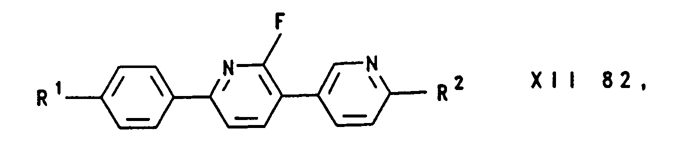 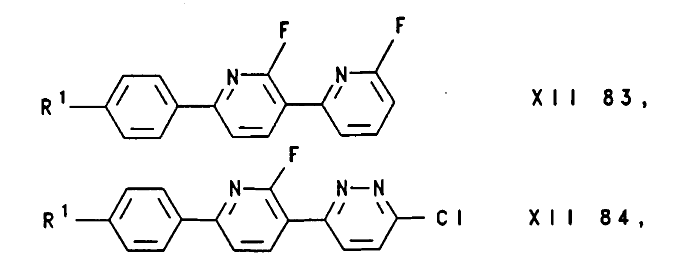 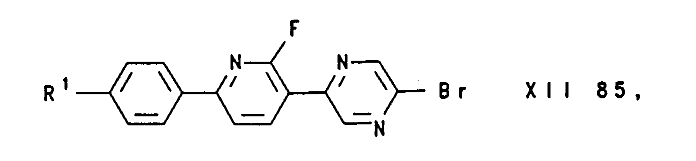 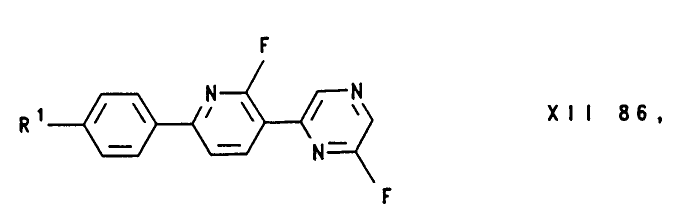 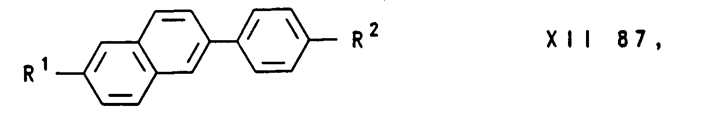 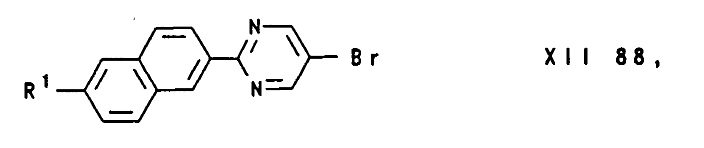 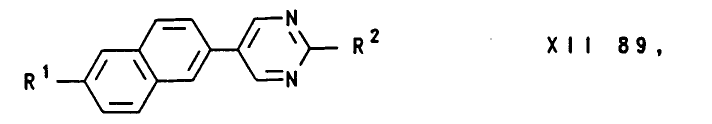 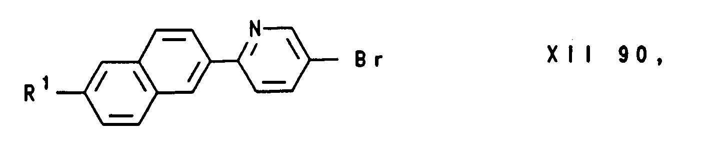 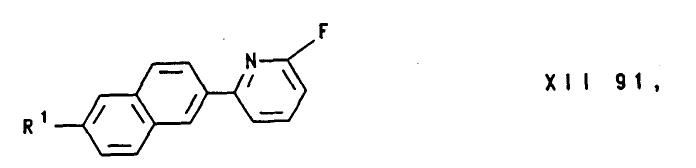 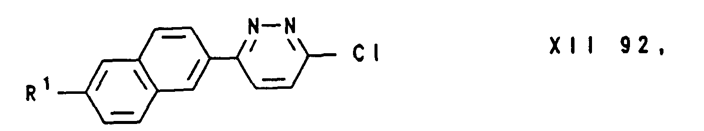 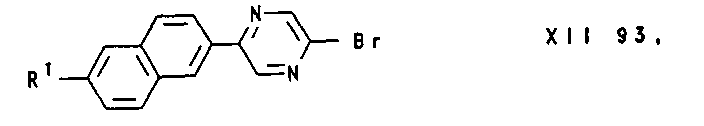 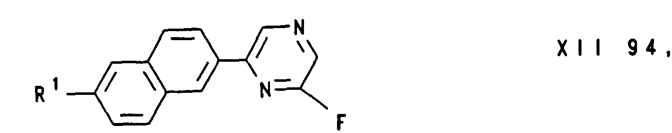 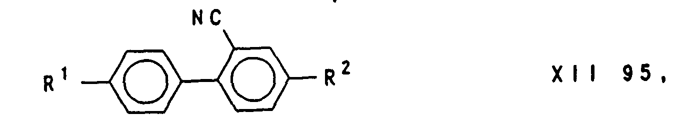 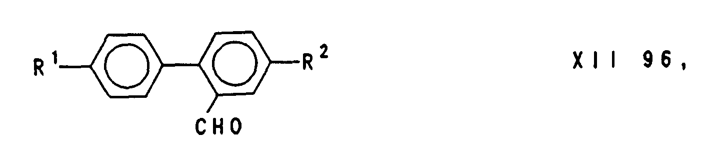 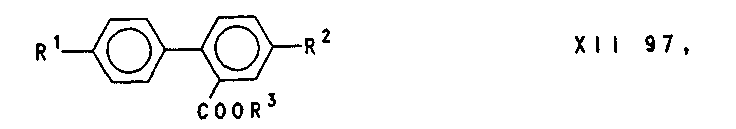 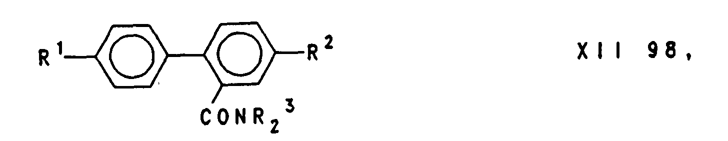 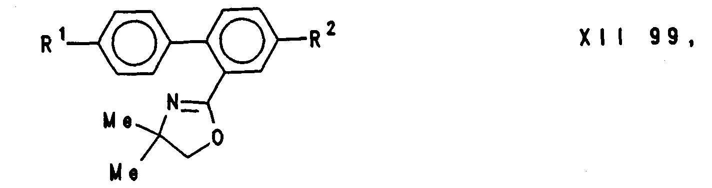 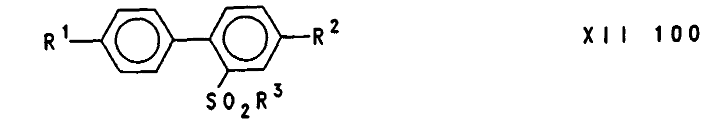 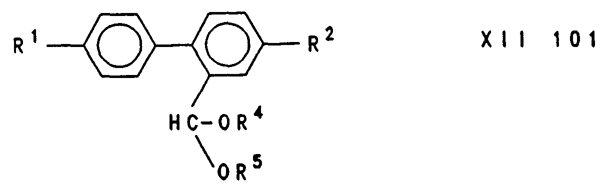 wobei R¹, R² und R³ Benzyloxy, H, Methyl, Ethyl, Propyl, Butyl, Pentyl, Hexyl, Heptyl, Octyl, Nonyl, Decyl, Undecyl, Dodecyl, Tridecyl, Tetradecyl und Pentadecyl, sowie Methoxy, Ethoxy, Propoxy, Butoxy, Pentoxy, Hexoxy, Heptoxy, Octoxy, Nonoxy, Decoxy, Undecoxy, Dodecoxy, Tridecoxy, Tetradecoxy und Pentadecoxy, R⁴, R⁵ Methyl, Ethyl, Propyl, Butyl, Pentyl, Hexyl, Heptyl, Octyl, Nonyl, Decyl, Undecyl, Dodecl, Tridecyl, Tetradecyl und Pentadecyl oder R⁴ und R⁵ zusammen auch -(CH₂)₂- oder -(CH₂)₃- bedeuten.

Die Verbindungen der Formel (XII) eignen sich zum Einsatz als flüssigkristalline Materialien oder können als Zwischenprodukte für die Herstellung weiterer flüssigkristalliner Verbindungen verwendet werden. Des weiteren werden Verbindungen der Formel (XII) als Vorprodukte für Pharmazeutika, Kosmetika, Fungizide, Herbizide, Insektizide, Farbstoffe, Detergenzien und Polymere, einschließlich als Zusatzstoffe derselben, eingesetzt.

Erfindungsgemäß hergestellte Verbindungen, wie sie beispielsweise durch die Formeln (XII 95) bis (XII 100) wiedergegeben werden, sind insbesondere wertvolle Vorstufen für Angiotensin II Inhibitoren (siehe z.B. Drugs of the Future 18 (1993) 428-432).

Die vorliegende Erfindung soll durch die nachfolgend beschriebenen Beispiele näher erläutert werden, ohne sie dadurch zu begrenzen. Die verwendeten Abkürzungen haben dabei folgende Bedeutung:
- Fp. =: Schmelzpunkt
- X =: kristallin
- S =: smektisch
- S_{C} =: smektisch C
- S_{A} =: smektisch A
- N =: nematisch
- I =: isotrop

### Beispiel 1

2-Brombenzonitril (3,1 g) und 2,75 g 4-Methylphenylboronsäure werden in 20 ml Toluol und 10 ml Ethanol gelöst. Man versetzt die Lösung mit 40,4 mg (1 Mol-%) Palladium(II)acetat und 4,47 g Natriumcarbonat in 10 ml Wasser. Anschließend gibt man 0,36 mmol 3,3',3"-Triphenylphosphinotrisulfonylnatrium (TPPTS) zu. Die Mischung wird 12 Stunden auf 80°C erhitzt. Nach beendeter Reaktion wird die Mischung auf Raumtemperatur abgekühlt und die organische Phase abgetrennt. Die wäßrige Phase wird dreimal mit Dichlormethan ausgeschüttelt. Die vereinigten organischen Phasen werden über Magnesiumsulfat getrocknet und anschließend werden die Lösungsmittel am Rotationsverdampfer eingedampft. Der Rückstand wird aus 50 ml n-Heptan kristallisiert. Ausbeute: 2,9 g 4-Methyl-2'-cyanobiphenyl, Schmelzpunkt 49°C.

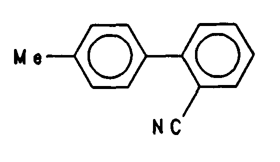

### Beispiel 2

2-Brombenzonitril (3,1 g) und 2,75 g 4-Methylphenylboronsäure werden in 20 ml Toluol und 10 ml Ethanol gelöst. Man versetzt die Lösung mit 4 mg (0,1 Mol-%) Palladium(II)acetat und 4,47 g Natriumcarbonat in 10 ml Wasser. Anschließend gibt man 0,036 mmol 3,3',3"-Triphenylphosphinotrisulfonylnatrium (TPPTS) zu. Die Mischung wird 18 Stunden auf 80°C erhitzt. Nach beendeter Reaktion wird die Mischung auf Raumtemperatur abgekühlt und die organische Phase abgetrennt. Die wäßrige Phase wird dreimal mit Dichlormethan ausgeschüttelt. Die vereinigten organischen Phasen werden über Magnesiumsulfat getrocknet und anschließend werden die Lösungsmittel am Rotationsverdampfer eingedampft. Der Rückstand wird aus 50 ml n-Heptan kristallisiert. Ausbeute: 2,7 g 4-Methyl-2'-cyanobiphenyl.

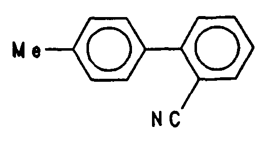

### Beispiel 3

2-Brombenzonitril (3,1 g) und 2,75 g 4-Methylphenylboronsäure werden in 20 ml Toluol und 10 ml Ethanol gelöst. Man versetzt die Lösung mit 40,4 mg (1 Mol-%) Palladium(II)acetat und 4,47 g Natriumcarbonat in 10 ml Wasser. Anschließend gibt man 0,18 mmol BINAS zu. Die Mischung wird 12 Stunden auf 80°C erhitzt. Nach beendeter Reaktion wird die Mischung auf Raumtemperatur abgekühlt und die organische Phase abgetrennt. Die wäßrige Phase wird dreimal mit Dichlormethan ausgeschüttelt. Die vereinigten organischen Phasen werden über Magnesiumsulfat getrocknet und anschließend werden die Lösungsmittel am Rotationsverdampfer eingedampft. Der Rückstand wird aus 50 ml n-Heptan kristallisiert. Ausbeute: 2,55 g 4-Methyl-2'-cyanobiphenyl.

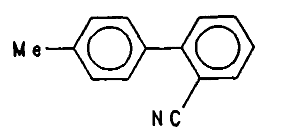

### Beispiel 4

2-Brombenzonitril (3,1 g) und 2,75 g 4-Methylphenylboronsäure werden in 20 ml Toluol und 10 ml Ethanol gelöst. Man versetzt die Lösung mit 4 mg (0,1 Mol-%) Palladium(II)acetat und 4,47 g Natriumcarbonat in 10 ml Wasser. Anschließend gibt man 0,018 mmol BINAS zu. Die Mischung wird 12 Stunden auf 80°C erhitzt. Nach beendeter Reaktion wird die Mischung auf Raumtemperatur abgekühlt und die organische Phase abgetrennt. Die wäßrige Phase wird dreimal mit Dichlormethan ausgeschüttelt. Die vereinigten organischen Phasen werden über Magnesiumsulfat getrocknet und anschließend werden die Lösungsmittel am Rotationsverdampfer eingedampft. Der Rückstand wird aus 50 ml n-Heptan kristallisiert. Ausbeute: 2,45 g 4-Methyl-2'-cyanobiphenyl.

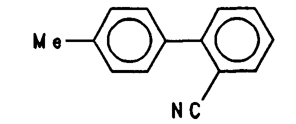

### Beispiel 5

10,43 g (0,044 Mol) 2,5-Dibrompyrimidin, 10 g (0,044 Mol) 4-(Phenylmethoxy)-benzolboronsäure, 98,8 mg (0,00044 Mol) Palladium(II)acetat, (0,001752 Mol) TPPTS und 9,3 g (0,0876 Mol) Natriumcarbonat werden in 100 ml Toluol, 50 ml Ethanol und 30 ml Wasser für 48 h auf 80°C erhitzt. Anschließend wird bei 80°C der Palladiumkatalysator durch Filtration vom Reaktionsgemisch abgetrennt. Die wäßrige Unterphase des Reaktionsgemisches wird bei 80°C abgetrennt bevor die organische Phase am Rotationsverdampfer von den Lösungsmitteln befreit und im Hochvakuum getrocknet wird. Das so erhaltene Rohprodukt wird aus Acetonitril (300 ml) kristallisiert, wonach 14,5 g (93 % Ausbeute, bezogen auf 2,5-Dibrompyrimidin) 5-Brom-2-[4-(phenylmethoxy)phenyl]pyrimidin (Gehalt nach HPLC 98 %) erhalten werden.

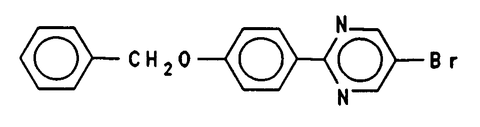

### Beispiel 6

2-Brombenzonitril (3,1 g) und 2,75 g 4-Methylphenylboronsäure werden in 20 ml Toluol und 10 ml Ethanol gelöst. Man versetzt die Lösung mit 108,5 mg (1 Mol-%) Bis(dibenzylidenaceton)palladium (Pd(dba)₂) und 4,47 g Natriumcarbonat in 10 ml Wasser. Anschließend gibt man 0,36 mmol 3,3',3"-Triphenylphosphinotrisulfonylnatrium (TPPTS) zu. Die Mischung wird 12 Stunden auf 80°C erhitzt. Nach beendeter Reaktion wird die Mischung auf Raumtemperatur abgekühlt und die organische Phase abgetrennt. Die wäßrige Phase wird dreimal mit Dichlormethan ausgeschüttelt. Die vereinigten organischen Phasen werden über Magnesiumsulfat getrocknet und anschließend werden die Lösungsmittel am Rotationsverdampfer eingedampft. Der Rückstand wird aus 50 ml n-Heptan kristallisiert. Ausbeute: 2,91 g 4-Methyl-2'-cyanobiphenyl, Schmelzpunkt 49°C.

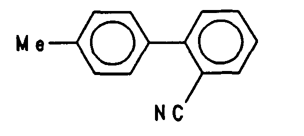

### Beispiel 7

2-Brombenzonitril (3,1 g) und 2,75 g 4-Methylphenylboronsäure werden in 20 ml Toluol und 10 ml Ethanol gelöst. Man versetzt die Lösung mit 10,4 mg (0,1 Mol-%) Pd(dba)₂ und 4,47 g Natriumcarbonat in 10 ml Wasser. Anschließend gibt man 0,036 mmol 3,3',3"-Triphenylphosphinotrisulfonylnatrium (TPPTS) zu. Die Mischung wird 18 Stunden auf 80°C erhitzt. Nach beendeter Reaktion wird die Mischung auf Raumtemperatur abgekühlt und die organische Phase abgetrennt. Die wäßrige Phase wird dreimal mit Dichlormethan ausgeschüttelt. Die vereinigten organischen Phasen werden über Magnesiumsulfat getrocknet und anschließend werden die Lösungsmittel am Rotationsverdampfer eingedampft. Der Rückstand wird aus 50 ml n-Heptan kristallisiert. Ausbeute: 2,69 g 4-Methyl-2'-cyanobiphenyl.

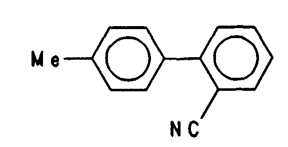

### Beispiel 8

2-Brombenzonitril (3,1 g) und 2,75 g 4-Methylphenylboronsäure werden in 20 ml Toluol und 10 ml Ethanol gelöst. Man versetzt die Lösung mit 103,5 mg (1 Mol-%) Pd(dba)₂ und 4,47 g Natriumcarbonat in 10 ml Wasser. Anschließend gibt man 0,18 mmol BINAS zu. Die Mischung wird 12 Stunden auf 80°C erhitzt. Nach beendeter Reaktion wird die Mischung auf Raumtemperatur abgekühlt und die organische Phase abgetrennt. Die wäßrige Phase wird dreimal mit Dichlormethan ausgeschüttelt. Die vereinigten organischen Phasen werden über Magnesiumsulfat getrocknet und anschließend werden die Lösungsmittel am Rotationsverdampfer eingedampft. Der Rückstand wird aus 50 ml n-Heptan kristallisiert. Ausbeute: 2,5 g 4-Methyl-2'-cyanobiphenyl.

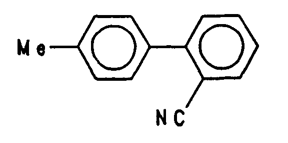

### Beispiel 9

2-Brombenzonitril (3,1 g) und 2,75 g 4-Methylphenylboronsäure werden in 20 ml Toluol und 10 ml Ethanol gelöst. Man versetzt die Lösung mit 10,4 mg (0,1 Mol-%) Pd(dba)₂ und 4,47 g Natriumcarbonat in 10 ml Wasser. Anschließend gibt man 0,018 mmol BINAS zu. Die Mischung wird 12 Stunden auf 80°C erhitzt. Nach beendeter Reaktion wird die Mischung auf Raumtemperatur abgekühlt und die organische Phase abgetrennt. Die wäßrige Phase wird dreimal mit Dichlormethan ausgeschüttelt. Die vereinigten organischen Phasen werden über Magnesiumsulfat getrocknet und anschließend werden die Lösungsmittel am Rotationsverdampfer eingedampft. Der Rückstand wird aus 50 ml n-Heptan kristallisiert. Ausbeute: 2,5 g 4-Methyl-2'-cyanobiphenyl.

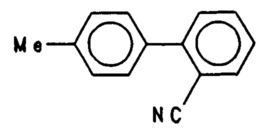

### Beispiel 10

10,43 g (0,044 Mol) 2,5-Dibrompyrimidin, 10 g (0,044 Mol) 4-(Phenylmethoxy)-benzolboronsäure, 253 mg (0,00044 Mol) Pd(dba)₂ (0,001752 Mol) TPPTS und 9,3 g (0,0876 Mol) Natriumcarbonat werden in 100 ml Toluol, 50 ml Ethanol und 30 ml Wasser für 48 h auf 80°C erhitzt. Anschließend wird bei 80°C der Palladiumkatalysator durch Filtration vom Reaktionsgemisch abgetrennt. Die wäßrige Unterphase des Reaktionsgemisches wird bei 80°C abgetrennt bevor die organische Phase am Rotationsverdampfer von den Lösungsmitteln befreit und im Hochvakuum getrocknet wird. Das so erhaltene Rohprodukt wird aus Acetonitril (300 ml) kristallisiert, wonach 14,7 g (93 % Ausbeute, bezogen auf 2,5-Dibrompyrimidin) 5-Brom-2-[4-(phenylmethoxy)phenyl]pyrimidin (Gehalt nach HPLC 98 %) erhalten werden.

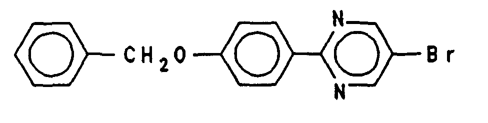

### Beispiel 11

Analog Beispiel 10 aus 27,7 g (100 mmol) 5-[1,3,2]-Dioxaborolan-2-yl-2-octyloxy-pyridin und 28,9 g (100 mmol) 5-Brom-2-octyloxypyrimidin, 0,288 g (0,5 mmol) Pd(dba)₂, 2 mmol TPPTS und 21,2 g (200 mmol) Natriumcarbonat. Nach Chromatographie an Kieselgel erhält man 39,6 g (96 %) Produkt. 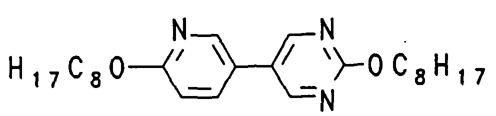 Phasenfolge: X 64 S_{C} 67 S_{A} 91 I

### Beispiel 12

Analog Beispiel 10 aus 24,3 g (100 mmol) 2-Brom-5-hexylpyrimidin und 21,9 g (100 mmol) 4-Hexyloxylbenzolboronsäure, 0,288 g (0,5 mmol) Pd(dba)₂, 2 mmol BINAS und 21,2 g (200 mmol) Natriumcarbonat. Nach Chromatographie an Kieselgel erhält man 32,4 g (95 %) Produkt 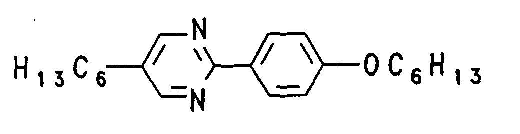 Phasenfolge: X₁ 15 X₂ 32 N 61 I

## Patentansprüche

1. Verfahren zur Herstellung mehrkerniger aromatischer Verbindungen durch Kreuzkupplung von aromatischen Borverbindungen mit aromatischen Halogenverbindungen oder Perfluoralkylsulfonaten unter Palladiumkatalyse in Gegenwart mindestens eines wasserlöslichen Komplexliganden in einem Wasser enthaltenden Reaktionsmedium, dadurch gekennzeichnet, daß das Reaktionsmedium eine wäßrige und eine organische Phase bildet und das Palladium in Form einer in der organischen Phase löslicher Palladiumverbindung aus der Gruppe Palladiumketonate, Palladiumacetylacetonate, Nitrilpalladiumhalogenide, Olefinpalladiumhalogenide, Palladiumhalogenide, Allylpalladiumhalogenide und Palladiumbiscarboxylate zugesetzt wird, und als wasserlöslicher Komplexligand mindestens eine Verbindung aus der Gruppe Phosphane, Phosphite, Phosphonigsäureester, Phosphinigsäureester, Phosphole, Bipyridine, Phenanthroline, Porphyrine und Alizarine verwendet wird.

2. Verfahren nach Anspruch 1, dadurch gekennzeichnet, daß eine mehrkernige aromatische Verbindung der Formel (XII) hergestellt wird,
R¹ (-A¹)ₖ(-M¹)ₗ-A²-A³(-M²)ₘ(-A⁴)ₙ-R² (XII)
worin die Symbole und Indizes folgende Bedeutungen haben:
R¹ und R² jeweils unabhängig voneinander Benzyloxy, H, F, Cl, Br, -NC, -CN, -CF₃, -OCF₃ oder ein geradkettiger, verzweigter (mit oder ohne asymmetrisches C-Atom) oder cyclischer Alkylrest mit 1 bis 18 C-Atomen, wobei auch eine ode zwei nicht benachbarte -CH₂-Gruppen durch -O-, -S-, -CO-, -CO-O-, -O-CO-, -CO-S-, -S-CO-, -O-CO-O-, -CH=CH-, -C≡C-, -SO₂-, CON(H,C₁-C₈-Alkyl), Cyclopropan-1,2-diyl oder -Si(CH₃)₂- ersetzt sein können, und wobei auch ein oder mehrere H-Atome des Alkylrestes durch F, Cl, Br oder CN substituiert sein können;
A¹ und A⁴ jeweils unabhängig voneinander 1,4-Phenylen, Pyrazin-2,5-diyl, Pyridazin-3,6-diyl, Pyridin-2,5-diyl, Pyrimidin-2,5-diyl, trans-1,4-Cyclohexylen, Naphthalin-2,6-diyl, wobei ein oder mehrere H-Atome gleich oder verschieden durch Substituenten L ersetzt sein können, wobei L die unter R¹ genannten Bedeutungen hat oder 4,4-Dimethylisoxazolin ist, und wobei eine oder zwei nicht benachbarte CH₂-Gruppen des Cyclohexylens durch -O- oder -S- ersetzt sein können, (1,3,4)-Thiadiazol-2,5-diyl, 1,3-Thiazol-2,4-diyl, 1,3-Thiazol-2,5- 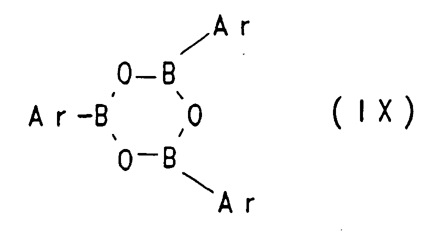 mit einer aromatischen Halogenverbindung oder einem Perfluoralkylsulfonat der Formel (XI) umsetzt,
X-A³(-M²)ₘ(-A⁴)ₙ-R² (XI)
wobei R², R³, A³, A⁴, M², m und n die in Formel (XII) angegebenen Bedeutungen haben und X Chlor, Brom, lod oder OSO₂-CₚF₂ₚ₊₁, worin p einen ganzzahligen Wert von 1 bis 10 darstellt, bedeutet.

3. Verfahren nach Anspruch 1, dadurch gekennzeichnet, daß eine in der organischen Phase lösliche Palladiumverbindung aus der Gruppe Bis(benzylidenaceton)palladium(0), Bis(benzylidenaceton)palladium(0)-Chloroformkomplex, Palladiumbisacetylacetonat, Palladiumdichlorid, Natriumtetrachloropalladat, Dichloro(dimethylsulfoxid)palladium(II), Bis(benzonitril)palladiumdichlorid, Bis(acetonitril)palladiumdichlorid, Palladium-II-acetat, Palladium-II-propionat, Palladium-II-butanoat und (1c,5c-Cyclooctadien)palladiumdichlorid verwendet wird.

4. Verfahren nach einem der Ansprüche 1 bis 3, dadurch gekennzeichnet, daß die organische Phase ein oder mehrere wasserunlösliche Lösungsmittel aus der Gruppe Kohlenwasserstoffe, Ether, höhere, mit Wasser nicht beliebig mischbare Alkohole, Ketone, Amide und Nitrile enthält.

5. Verfahren nach einem der Ansprüche 1 bis 4, dadurch gekennzeichnet, daß die wäßrige Phase der Reaktionsmischung ein wassermischbares organisches Colsolvens aus der Gruppe Nitrile, Amide und niedere Alkohole enthält.

6. Verfahren nach einem der Ansprüche 1 bis 5, dadurch gekennzeichnet, daß im Verfahren eine Base und zwar mindestens eine Verbindung aus der Gruppe Alkali und Erdalkalimetallhydroxide, Alkali- und Erdalkalimetallcarbonate, Alkalimetallhydrogencarbonate, Alkali- und Erdalkalimetallacetate, Alkali- und Erdalkalimetallalkloholate und primäre, sekundäre und tertiäre Amine verwendet wird.

## Claims

1. A process for preparing polycyclic aromatic compounds by cross-coupling aromatic boron compounds with aromatic halogen compounds or perfluoroalkylsulfonates under palladium catalysis in the presence of at least one water-soluble complexing ligand in a water containing reaction medium, characterised in that the reaction medium forms an aqueous and an organic phase and the palladium is added in the form of a palladium compound selected from the group consisting of palladium ketonates, palladium acetylacetonates, (nitrile)-palladium halides, (olefin)palladium halides, palladium halides, allylpalladium halides and palladium biscarboxylates and soluble in the organic phase, and wherein at least one compound selected from the group consisting of phosphines, phosphites, phosphonous esters, phosphinous esters, phospholes, bipyridines, phenanthrolines, porphyrins and alizarins is used as a water-soluble complexing ligand.

2. The process as claimed in claim 1, wherein a polycyclic aromatic compound of the formula (XII),
R¹(-A¹)ₖ(-M¹)ₗ-A²-A³(-M²)ₘ(-A⁴)ₙ-R² (XII)
where the symbols and indices have the following meanings:
R¹ and R² are each, independently of one another, benzyloxy, H, F, Cl, Br, -NC, -CN, -CF₃, -OCF₃ or a straight-chain, branched (with or without an asymmetric carbon atom) or cyclic alkyl radical having from 1 to 18 carbon atoms, where one or two nonadjacent -CH₂- groups can also be replaced by -O-, -S-, -CO-, -CO-O-, -O-CO-, -CO-S-, -S-CO-, -O-CO-O-, -CH=CH-, -C≡C-, -SO₂-, CON(H,C₁-C₈-alkyl), cyclopropane-1,2-diyl or -Si(CH₃)₂- and where one or more hydrogen atoms of the alkyl radical can also be replaced by F, Cl, Br or CN;
A¹ and A⁴ are each, independently of one another, 1,4-phenylene, pyrazine-2,5-diyl, pyridazine-3,6-diyl, pyridine-2,5-diyl, pyrimidine-2,5-diyl, trans-1,4-cyclohexylene, naphthalene-2,6-diyl, where one or more hydrogen atoms can be replaced by identical or different substituents L, where L has one of the meanings given under R¹ or is 4,4-dimethylisoxazoline, and where one or two nonadjacent -CH₂ groups of the cyclohexylene can be replaced by -O- or -S-, or 1,3,4-thiadiazol-2,5-diyl, 1,3-thiazol-2,4-diyl, 1,3-thiazol-2,5-diyl, thiophene-2,4-diyl, thiophene-2,5-diyl, piperazine-1,4-diyl, piperazine-2,5-diyl, piperidine-1,4-diyl, bicyclo[2.2.2]octane-1,4-diyl, 1,3-dioxaborinane-2,5-diyl or trans-decalin-2,6-diyl;
A² and A³ are each, independently of one another, 1,4-phenylene, pyrazine-2,5-diyl, pyridazine-3,6-diyl, pyridine-2,5-diyl, pyrimidine-2,5-diyl, naphthalene-2,6-diyl, where one or more hydrogen atoms can be replaced by identical or different substituents L, where L has one of the meanings given under R¹, is 4,4-dimethylisoxazoline or, in the case of A³, is also -CHO, or 1,3,4-thiadiazol-2,5-diyl, 1,3-thiazol-2,4-diyl, 1,3-thiazol-2,5-diyl, thiophene-2,4-diyl or thiophene-2,5-diyl;
M¹ and M² are each, independently of one another, -O-, -S-, -CO-, -CO-O-, -O-CO-, -CO-S, -S-CO-, -O-CO-O-, -CH₂-O-, -OCH₂-, -CH₂CH₂-, -CH=CH-, -C≡C-, -CH(CN)-CH₂-, -CH₂-CH(CN)-, -CH=N-, -N=CH-, -CH₂CH₂CH₂-O-, -OCH₂CH₂CH₂-, -CH₂CH₂CO-O-, -O-COCH₂CH₂-;
k, I, m, n are each, independently of one another, zero or one; is prepared by reacting an aromatic boron compound of the formula (VII),
R¹(-A¹)ₖ(-M¹)ₗ-A²-BQ₁Q₂ (VII)
where R¹, A¹, A², M¹, k and I are as defined for formula (XII),
Q₁, Q₂ are identical or different and are -OH, C₁-C₄-alkoxy, C₁-C₄-alkyl, phenyl, which may be unsubstituted or substituted by C₁-C₄-alkyl, C₁-C₄-alkoxy or halogen, or are halogen, or Q₁ and Q₂ together form a C₁-C₄-alkylenedioxy group, a methylene group which may be unsubstituted or substituted by one or two C₁-C₄-alkyl groups, or Q₁ and Q₂ and the boron atom together are part of a boroxine ring of the 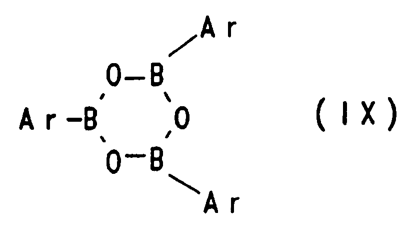 with an aromatic halogen compound or a perfluoroalkylsulfonate of the formula (XI),
X-A³(-M²)ₘ(-A⁴)ₙ-R² (XI)
where R², R³, A³, A⁴, M², m and n are as defined for formula (XII) and X is chlorine, bromine, iodine or OSO₂-CₚF₂ₚ₊₁, where p has an integral value from 1 to 10.

3. The process as claimed in claim 1, wherein a palladium compound soluble in the organic phase is selected from the group consisting of bis(benzylideneacetone)palladium(0), bis(benzylideneacetone)palladium(0)-chloroform complex, palladium bisacetylacetonate, palladium dichloride, sodium tetrachloropalladate, dichloro(dimethyl sulfoxide)palladium(II), bis(benzonitrile)palladium dichloride, bis(acetonitrile)palladium dichloride, palladium(II) acetate, palladium(ll) propionate, palladium(II)butanoate and (1c,5c-cyclooctadiene)palladium dichloride.

4. The process as claimed in one of claims 1 to 3, wherein the organic phase comprises one or more water-insoluble solvents selected from the group consisting of hydrocarbons, ethers, higher alcohols which are not completely miscible with water, ketones, amides and nitriles.

5. The process as claimed in one of claims 1 to 4, wherein the aqueous phase of the reaction mixture comprises a water-miscible organic cosolvent selected from the group consisting of nitriles, amides and lower alcohols.

6. The process as claimed in one of claims 1 to 5, wherein in the process a base which is at least one compound selected from the group consisting of alkali metal and alkaline earth metal hydroxides, alkali metal and alkaline earth metal carbonates, alkali metal hydrogencarbonates, alkali metal and alkaline earth metal acetates, alkali metal and alkaline earth metal alcoholates and primary, secondary and tertiary amines is used as base.

## Revendications

1. Procédé pour la préparation de composés aromatiques polycycliques par couplage croisé de composés aromatiques du bore avec des composés aromatiques halogénés ou des sulfonates de perfluoroalkyle sous catalyse au palladium en présence d'au moins un ligand de complexe hydrosoluble dans un milieu réactionnel contenant de l'eau, caractérisé en ce que, le milieu réactionnel forme une phase aqueuse et une phase organique et en ce que le palladium est ajouté sous la forme d'un composé de palladium soluble dans la phase organique choisi parmi les cétonates de palladium, les acétylacétonates de palladium, les halogénures de palladium nitrile, les halogénures de palladium oléfine, les halogénures de palladium, les halogénures de palladium allyle et les biscarboxylates de palladium, et en ce qu'on utilise comme ligand hydrosoluble du complexe au moins un composé choisi parmi les phosphanes, phosphites, phosphonates, phosphinates, phosphols, bipyridines, phénanthrolines, porphyrines et alizarines.

2. Procédé selon la revendication 1, caractérisé en ce que, l'on prépare un composé aromatique polycyclique de formule (XII),
R¹(-A¹)ₖ(-M¹)ₗ-A²-A³(-M²)ₘ(-A⁴)ₙ-R² (XII)
dans laquelle les symboles et indices ont les significations suivantes :
R¹ et R² représentent respectivement indépendamment l'un de l'autre un groupe benzyloxy, un atome d'hydrogène, de fluor, de chlore, de brome, un groupe -NC, -CN, -CF₃-, -OCF₃- ou un groupe alkyle à chaîne linéaire, ramifiée (avec ou sans atome de carbone asymétrique) ou cyclique ayant de 1 à 18 atomes de carbone, dans lequel un ou plusieurs groupes -CH₂- non voisins peuvent aussi être remplacés par -O-, -S-, -CO-, -CO-O-, -O-CO-, -CO- S-, -S-CO-, -O-CO-O-, -CH=CH-, -C=C-, -SO₂-, CON(H, alkyle en C₁ à C₈), cyclopropan-1,2-diyle ou -Si(CH₃)₂-, et dans lequel un ou plusieurs atomes d'hydrogène du groupe alkyle peuvent également être substitués par F, Cl, Br ou CN;
A¹ et A² représentent respectivement indépendamment l'un de l'autre un groupe 1,4-phénylène, pyrazin-2,5-diyle, pyridazin-3,6-diyle, pyridin-2,5-diyle, pyrimidin-2,5-diyle, trans-1,4-cyclohexylène, naphtalèn-2,6-diyle, dans lequel un ou plusieurs atomes d'hydrogène identiques ou différents peuvent être substitués par des substituants L, L ayant les significations indiquées pour R¹ ou étant un groupe 4,4-diméthylisoxazoline, et dans lequel un ou deux groupes -CH₂- non voisins du groupe cyclohexylène peuvent être remplacés par -O- ou -S-, un groupe (1,3,4)-thiadiazol-2,5-diyle, 1,3-thiazol-2,4-diyle, 1,3-thiazol-2,5-diyle, thiophèn-2,4-diyle, thiophèn-2,5-diyle, pipérazin-1,4-diyle, pipérazin-2,5-diyle, pipéridin-1,4-diyle, bicyclo[2.2.2]-octan-1,4-diyle, 1,3-dioxaborinan-2,5-diyle ou trans-décalin-2,6-diyle;
A² et A³ représentent respectivement indépendamment l'un de l'autre un groupe 1,4-phénylène, pyrazin-2,5-diyle, pyridazin-3,6-diyle, pyridin-2,5-diyle, pyrimidin-2,5-diyle, naphtalèn-2,6-diyle, dans lequel un ou plusieurs atomes d'hydrogène identiques ou différents peuvent être substitués par des substituants L, L ayant les significations indiquées pour R¹, étant un groupe 4,4-diméthylisoxazoline, ou étant dans le cas de A³ également un groupe -CHO, 1,3,4-thiadiazol-2,5-diyle, 1,3-thiazol-2,4-diyle, 1,3-thiazol-2,5-diyle, thiophèn-2,4-diyle ou thiophèn-2,5-diyle;
M¹ et M² représentent respectivement indépendamment l'un de l'autre -O-, -S-, -CO-, -CO-O-, O-CO-, -CO-S, -S-CO-, -O-CO-O-, -CH₂-O-, -OCH₂-, -CH₂CH₂-, -CH=CH-, -C≡C- -CH(CN)-CH₂-, -CH₂-CH(CN)-, -CH=N-, -N=CH-, -CH₂CH₂CH₂-O-, -OCH₂CH₂CH₂-, -CH₂CH₂CO-O-, -O-COCH₂CH₂-;
k, l, m, n, représentent respectivement indépendamment les uns des autres zéro ou un;
dans lequel on met à réagir un composé aromatique du bore de formule (VII),
R¹(-A¹)ₖ(-M¹)ₗ-A²-BQ₁Q₂ (VII)
dans laquelle R¹, A¹, A², M¹, k et l ont les significations indiquées dans la formule (XII),
Q¹ et Q² identiques ou différents représentent -OH, un groupe alcoxy en C₁ à C₄, alkyle en C₁ à C₄, phényle qui peut être éventuellement substitué par un groupe alkyle en C₁ à C₄, alcoxy en C₁ à C₄ ou un atome d'halogène, ou représentent un atome d'halogène ou Q¹ et Q² forment ensemble un groupe dioxyalkylène en C₁ à C₄, un groupe méthylène, qui peut être éventuellement substitué par un ou deux groupes alkyle en C₁ à C₄, ou Q¹ et Q² et l'atome de bore sont ensemble une partie d'un composé cyclique du bore de formule (IX) : 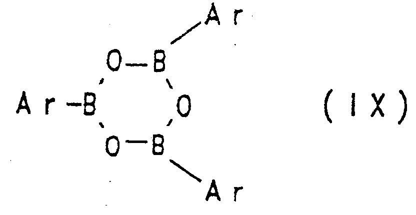
avec un composé halogéné aromatique ou un sulfonate de perfluoroalkyle de formule (XI),
X-A³(-M²)ₘ(-A⁴)ₙ-R² (XI)
dans laquelle R², R³, A³, A⁴, M², m et n ont les significations indiquées à la formule (XII) et X représente un atome de chlore, de brome, d'iode ou un groupe OSO₂-CₚF₂ₚ₊₁, dans lequel p représente un nombre entier de 1 à 10.

3. Procédé selon la revendication 1, caractérisé en ce que, l'on utilise un composé du palladium soluble dans la phase organique choisi parmi le Bis(benzylidènacéton)palladium(0), le complexe avec le chloroforme du Bis(benzylidènacéton)palladium(O), le bisacétylacétonate de palladium, le dichlorure de palladium, le tétrachloropalladate de sodium, le Dichloro(diméthylsulfoxide)palladium(II), le Bis(benzonitril)palladiumdichlorure, le Bis(acétonitril)palladiumdichlorure, l'acétate de palladium-II, le propionate de palladium-II, le butanoate de palladium-II et le dichlorure de (1c,5c-cyclooctadièn)palladium.

4. Procédé selon l'une des revendications 1 à 3, caractérisé en ce que, la phase organique contient un ou plusieurs solvants insolubles dans l'eau choisis parmi les hydrocarbures, les éthers, les alcools supérieurs, non facilement miscibles à l'eau, les cétones, les amides et les nitriles.

5. Procédé selon l'une des revendications 1 à 4, caractérisé en ce que, la phase aqueuse du mélange réactionnel contient un co-solvant organique miscible à l'eau choisi parmi les nitriles, les amides et les alcools inférieurs.

6. Procédé selon l'une des revendications 1 à 5, caractérisé en ce que, l'on utilise dans le procédé une base et en cela au moins un composé choisi parmi les hydroxydes de métaux alcalins et alcalino-terreux, les carbonates de métaux alcalins et alcalino-terreux, les hydrogénocarbonates de métaux alcalins, les acétates de métaux alcalins et alcalino-terreux, les alcoolates de métaux alcalins et alcalino-terreux, et les amines primaires, secondaires et tertiaires.
